# EUROPEAN PATENT APPLICATION

(11) **EP 2 078 747 A1**
(43) Date of publication of application: **15.07.2009**
(21) Application number: 07832869.7
(22) Date of filing: 30.11.2007
(51) Int. Cl.: C12N 15/00, C12N 15/09, C12P 19/34, C12Q 1/68

(54) **PRIMER SET FOR AMPLIFICATION OF NAT2 GENE, REAGENT FOR AMPLIFICATION OF NAT2 GENE COMPRISING THE SAME, AND USE OF THE SAME**

(30) Priority: 30.11.2006 JP 2006322956; 07.09.2007 JP 2007232612
(71) Applicant: Arkray, Inc., Kyoto-shi, Kyoto 601-8045 (JP)
(72) Inventor: HIRAI, Mitsuharu, Kyoto-shi Kyoto 601-8045 (JP); MAJIMA, Satoshi, Kyoto-shi Kyoto 601-8045 (JP)
(74) Representative: Behnisch, Werner
(86) International application number: PCT/JP2007/073204
(87) International publication number: WO 2008/066161

(57) **Abstract**

Primer sets for amplifying target regions containing sites to be detected in the NAT2 gene by a gene amplification method are provided, wherein the primer sets can amplify the regions specifically. Three pairs of primer sets are used including forward primers consisting of the base sequences of SEQ ID NOs: 7, 33, and 60 as well as reverse primers consisting of the base sequences of SEQ ID NOs: 18, 48 and 81, respectively. The use of these primer sets makes it possible to amplify three target regions including parts where three types of polymorphisms (NAT2*5, NAT2*6, and NAT2*7) of the NAT2 gene are generated, respectively, in the same reaction solution at the same time.

## Description

### Technical Field

The present invention relates to primer sets for amplifying the NAT2 gene, reagents for amplifying the NAT2 gene containing the same, and the uses thereof.

### Background Art

N-acetyltransferase (NATs) is an enzyme that is involved in a metabolic pathway in which the aryl amine of an aromatic amine and a heterocyclic amine is metabolized into nontoxic and stable substance by N-conjugation. Among them, NAT2, a subtype of NATs, is involved in: a detoxification process of homocyclic and heterocyclic aryl amine and hydrazine-like drugs such as isoniazid (INH), sulfamethazine, other sulfonamides, procainamide, hydralazine, caffeine, dapsone, etc.; and an activation of expressive biological substances and environment substances such as 2-aminofluorene, 4-aminobiphenyl, benzidine, β-naphthylamine, heterocyclic aryl amines existed in a pyrolytic substance of protein, etc. It is known that NAT2 expresses polymorphism and 19 types of polymorphism are known with respect to humans. Among these polymorphisms, with respect to NAT2*5 type (NAT2*5A to 5D), T (thymine) in the 341 position in mRNA of NAT2 gene is mutated to C (cytosine), with respect to NAT2*6 type (NAT2*6A and NAT2*6B), G (guanine) in the 590 position in the mRNA is mutated to A (adenine), and with respect to NAT2*7 type (NAT2*7A and NAT2*7B), G (guanine) in the 857 position in the mRNA is mutated into A (adenine).

It is known that when a patient with such mutation takes INH, which is an antiantagonist, hepatic dysfunction may occur. This is because NAT2 activity is changed due to NAT2 gene mutation, sufficient N-acetylation is thus not performed, and generation of hydrazine having high hepatotoxicity is increased. Besides that, it is known that a polymorphism of NAT2 gene is related to side effects of the aforementioned procainamide and sulfasalazine. Accordingly, confirmation of the polymorphism of NAT2 gene of the patient is very important for avoiding the side effect and administering an appropriate medication. Particularly, with respect to NAT2, it is important to confirm a plurality of polymorphisms (NAT2*5, NAT2*6, and NAT2*7).

On the other hand, detection of a point mutation, a so-called single nucleotide polymorphism (SNP), is employed widely as a method of analyzing, at the gene level, for example, the causes of all types of diseases and the individual differences in disease liability (susceptibility to diseases) and in drug action. Examples of the common methods of detecting a point mutation include: (1) a direct sequencing method in which the region corresponding to a sequence to be detected in a target DNA of a sample is amplified by a polymerase chain reaction (PCR) and all the gene sequences are analyzed, (2) a RFLP analysis in which the region corresponding to a sequence to be detected in a target DNA of a sample is amplified by PCR, the amplification product thus obtained is cut with a restriction enzyme whose cleaving action differs depending on the presence or absence of the target mutation in the sequence to be detected and then is electrophoresed, and thereby typing is performed, and (3) the ASP-PCR method in which PCR is performed using a primer with a target mutation located at the 3'-end region and the mutation is judged depending on the presence or absence of amplification.

However, since these methods require, for example, purification of DNA extracted from a sample, electrophoresis, and a treatment with a restriction enzyme, they take time and cost. Furthermore, after PCR is performed, it is necessary to open the reaction container once. Accordingly, there is a possibility that the amplification product may contaminate the next reaction system and thereby the analysis accuracy may be deteriorated. Moreover, since it is difficult to automate, a large amount of samples cannot be analyzed. Further, the aforementioned ASP-PCR method (3) is less specific, which also is a problem.

Because of these problems, recently, a method of analyzing the melting temperature (Tm) of double-stranded nucleic acid formed of a probe and target nucleic acid is used as a method of detecting a point mutation. Since such a method is performed through, for example, Tm analysis or analysis of the melting curve of the double strand, it is referred to as melting curve analysis. This method is described below. That is, first, a probe complementary to a sequence to be detected containing a target point mutation is used to form a hybrid (double-stranded DNA) between the aforementioned probe and a target single-stranded DNA contained in a detection sample. Subsequently, this hybridization product is heat-treated, and dissociation (melting) of the hybrid accompanying the temperature rise is detected by a change in a signal such as absorbance. The Tm value is then determined based on the result of the detection and the presence or absence of any point mutation is judged accordingly. The higher the homology of the hybridization product, the higher the Tm value, and the lower the homology, the lower the Tm value. Therefore the Tm value (reference value for assessment) is determined beforehand with respect to the hybridization product between the sequence to be detected containing a point mutation and a probe complementary thereto, and then the Tm value (measured value) of the hybridization product between the target single-stranded DNA contained in the detection sample and the aforementioned probe is measured. When the measured value is comparable to the reference value, it is considered as matching, that is, it can be judged that a point mutation is present in the target DNA. On the other hand, when the measured value is lower than the reference value, it is considered as mismatching, that is, it can be judged that no point mutation is present in the target DNA. Furthermore, according to this method, it also is possible to automate gene analysis.

However, such a detection method using Tm analysis also has a problem in that a region including a site to be detected must be able to be amplified specifically and efficiently in PCR. Particularly, many isozymes are present in NAT and the sequences for coding them also are very similar to each other. Accordingly, there is a possibility that genes coding isozymes other than NAT2 also are amplified in PCR. Furthermore, when other isozyme-coding genes also have been amplified as described above, it may cause a decrease in reliability of the analysis result in the analysis of a particular polymorphism (NAT2*5, NAT2*6, or NAT2*7) of the NAT2 gene (Nonpatent Document 1 or 2). Moreover, as described above, since analysis of one sample is accompanied by a considerable amount of time and energy, it is not practical to analyze many samples, which also is a problem.
[Nonpatent Document 1] PMID: 8102908 Jpn J Hum Genet. 1993 Jun; 38(2):163-8.
[Nonpatent Document 2] PMID:10507782 Br J Cancer. 1999 Oct; 81(3): 537-41.

### Disclosure of Invention

Hence, the present invention is intended to provide primer sets for specifically amplifying a target region in the NAT2 gene by a gene amplification method.

In order to achieve the aforementioned object, a primer set of the present invention is a primer set for amplifying the NAT2 gene by a gene amplification method, wherein the primer set includes at least one selected from the group consisting of the following primer sets (1) to (3):
Primer set (1):
   a primer set of a pair of primers including a forward primer composed of the following oligonucleotide (F1) and a reverse primer composed of the following oligonucleotide (R1):
      (F1): at least one oligonucleotide having a sequence identical to that of a region extending from guanine (G) at base 1038 to be considered as the first base to any one of the 20^{th} to 32^{nd} bases in the direction toward the 5' end in the base sequence of SEQ ID NO: 1, with the guanine (G) being the 3' end, and
      (R1): at least one oligonucleotide complementary to a region extending from cytosine (C) at base 1096 to be considered as the first base to any one of the 17^{th} to 24^{th} bases in the direction toward the 3' end in the base sequence of SEQ ID NO: 1, with guanine (G) complementary to the cytosine (C) at base 1096 being the 3' end,
Primer set (2):
   a primer set of a pair of primers including a forward primer composed of the following oligonucleotide (F2) and a reverse primer composed of the following oligonucleotide (R2):
      (F2): at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
      (R2): at least one oligonucleotide selected from:
   at least one oligonucleotide complementary to a region extending from guanine (G) at base 1355 to be considered as the first base to any one of the 25^{th} to 40^{th} bases in the direction toward the 3' end in the base sequence of SEQ ID NO: 1, with cytosine (C) complementary to the guanine (G) at base 1355 being the 3' end, and
   at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end, and
Primer set (3):
   a primer set of a pair of primers including a forward primer composed of the following oligonucleotide (F3) and a reverse primer composed of the following oligonucleotide (R3):
      (F3): at least one oligonucleotide selected from:
   at least one oligonucleotide having a sequence identical to that of a region extending from thymine (T) at base 1556 to be considered as the first base to any one of the 21^{st} to 40^{th} bases in the direction toward the 5' end in the base sequence of SEQ ID NO: 1, with the thymine (T) being the 3' end, and
   at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
      (R3): at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end.

A reagent for amplifying a gene of the present invention is a reagent for amplifying the NAT2 gene by a gene amplification method, wherein the reagent includes the primer set for amplifying the NAT2 gene of the present invention.

A method of manufacturing an amplification product of the present invention is a method of manufacturing an amplification product of the NAT2 gene by a gene amplification method, wherein the method includes the following step (I):
(I) amplifying the NAT2 gene in a reaction solution using a primer set for amplifying the NAT2 gene according to the present invention, with nucleic acid contained in a sample being used as a template.

A polymorphism analysis method of the present invention is a method of analyzing a polymorphism of three sites to be detected in the NAT2 gene, wherein the method includes the following steps (i) to (iv):
(i) amplifying a region including a site to be detected in the NAT2 gene in a reaction solution by a method of manufacturing an amplification product of the present invention,
(ii) preparing a reaction solution that contains the amplification product obtained in step (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

The primer set of the present invention makes it possible to specifically and efficiently amplify a target region in a reaction solution, with the target region including the site where a polymorphism to be detected (NAT2*5, NAT2*6, or NAT2*7) is generated in the NAT2 gene. Accordingly, the time and cost can be reduced, which is different from the conventional methods described above. Furthermore, as described above, since a region including a site to be detected where a specific polymorphism of the NAT2 gene is generated can be amplified specifically, for example, further the use of a probe complementary to a sequence to be detected including the site to be detected makes it possible to perform Tm analysis by directly using the aforementioned reaction solution to type the polymorphism. Moreover, since amplification and typing can be performed with one reaction solution, it is also possible to automate the operation. Since the use of the primer set of the present invention allows a pretreatment to be omitted even in the case of, for example, a contaminated sample (for instance, whole blood or oral mucosa), the amplification reaction can be carried out more quickly and simply. Furthermore, since the use of the primer set of the present invention allows the amplification reaction to be carried out with higher amplification efficiency as compared to the conventional case, the amplification reaction time also can be shortened. Thus, according to the primer set of the present invention and a reagent including the same as well as the method of manufacturing an amplification product and a polymorphism analysis method, in each of which the primer set and the reagent are used, polymorphisms in the NAT2 gene can be analyzed quickly and simply, and it therefore can be said that they are very effective in the field of medicine.

### Brief Description of Drawings

FIG. 1 shows graphs indicating the results of Tm analysis in Example 1 of the present invention.
FIG. 2 shows graphs indicating the results of Tm analysis in Example 2 of the present invention.

### Best Mode for Carrying Out the Invention

### < Primer set for amplifying NAT2 gene>

As described above, the primer set for amplifying the NAT2 gene of the present invention is characterized by including at least one primer set selected from the group consisting of the aforementioned primer sets (1) to (3). The inclusion of at least one of the primer sets makes it possible, for example, to specifically amplify a specific target region in the NAT2 gene.

The primer set for amplifying the NAT2 gene of the present invention may include, for example, one of the aforementioned primer sets (1) to (3) or may include two or all of the primer sets (1) to (3). As described later, the target region that can be amplified specifically with the primer set (1) is a region including a site where the polymorphism NAT2*5 is generated in the NAT2 gene; the target region that can be amplified specifically with the primer set (2) is a region including a site where the polymorphism NAT2*6 is generated in the NAT2 gene; and the target region that can be amplified specifically with the primer set (3) is a region including a site where the polymorphism NAT2*7 is generated in the NAT2 gene.

As described above, since these three types of polymorphisms in the NAT2 gene are known as polymorphisms that affect drug metabolism, it is considered to be important to examine not only one of them but two or all of the three types of polymorphisms. However, the conventional methods have a problem in that a plurality of sequences cannot be analyzed in one reaction system. Conceivably, as described above, this is because the many isozymes exist in a NAT and thereby genes coding isozymes other than NAT2 also are amplified in PCR. Accordingly, in order to examine two or all of the three types of polymorphisms (NAT2*5, NAT2*6, and NAT2*7) in the NAT2 gene, it is necessary that the regions including the sites where the respective polymorphisms are generated are amplified in separate reaction systems, respectively, and the resultant amplification products are analyzed separately. Thus, with the conventional methods, it is very difficult to use only the NAT2 gene selected from the NAT genes as a template and to amplify specifically only two or three types of target regions including the sites where polymorphisms are generated, respectively, in the NAT2 gene. Furthermore, since such analysis of even one sample is accompanied by a considerable amount of work, there is a problem in that the analysis of many samples is not practical. On the contrary, according to the primer set for amplifying the NAT2 gene of the present invention, even in the case where two or all of the three types of the primer sets (1) to (3) are included, the respective target regions can be amplified in the same reaction solution simultaneously and specifically. Accordingly, the time and cost can be reduced, which is different from the aforementioned conventional methods. Furthermore, since two or three target regions are amplified specifically in the same reaction solution as described above, for example, the use of a probe complementary to a sequence to be detected in each target region makes it possible to perform Tm analysis directly using the aforementioned reaction solution to type each of the two or three types of polymorphisms. As described above, since two or three types of polymorphisms in the NAT2 gene can be analyzed in the same reaction solution, it is suitable for the primer set for amplifying the NAT2 gene of the present invention not only to include one of the primer sets (1) to (3) but also to include two or three of them. When not only one target region but also two or three target regions are amplified simultaneously using such a primer set for amplifying the NAT2 gene, polymorphisms in the NAT2 gene can be analyzed more efficiently as compared to the conventional cases.

Hereinafter, a forward primer also may be referred to as "F primer" and a reverse primer as "R primer".

As described above, the primer set (1) is a primer set of a pair of primers including a forward primer composed of the following oligonucleotide (F1) and a reverse primer composed of the following oligonucleotide (R1).
(F1): at least one oligonucleotide having a sequence identical to that of a region extending from guanine (G) at base 1038 to be considered as the first base to any one of the 20^{th} to 32^{nd} bases in the direction toward the 5' end in the base sequence of SEQ ID NO: 1, with the guanine (G) being the 3' end, and
(R1): at least one oligonucleotide complementary to a region extending from cytosine (C) at base 1096 to be considered as the first base to any one of the 17^{th} to 24^{th} bases in the direction toward the 3' end in the base sequence of SEQ ID NO: 1, with guanine (G) complementary to the cytosine (C) at base 1096 being the 3' end,

The base sequence indicated in SEQ ID NO: 1 is a DNA sequence of the Human h NAT allele 1-2 gene for arylamine N-acetyltransferase and, for example, has been registered at NCBI under the accession No. D10870.

The primer set (1) is a primer set for amplifying a DNA strand including a region from base 1039 to base 1095 in SEQ ID NO: 1, as well as a strand complementary thereto. Base 1063 in this region (i.e. base 1063 in SEQ ID NO: 1) is known for the presence of a point mutation (1063T, 1063C) that affects the function of NAT2, and the polymorphism thereof is NAT2*5 described above. In the present invention, the polymorphism of this site can be indicated as 1063T/T or 1063C/C in the case of homozygote and as 1063T/C in the case of heterozygote. Further, since base 1063 in SEQ ID NO: 1 corresponds to base 341 in mRNA of the NAT2 gene, as the polymorphism NAT2*5, it can also be indicated, for example, as 341T/T, 341C/C, or 341T/C. Hereinafter, this primer set (1) also may be referred to as a "primer set for NAT2*5". When only the polymorphism NAT2*5 is to be analyzed, it is sufficient to use only the primer set for NAT2*5.

The F1 primer and R1 primer of the primer set (1) can be any primers, as long as the base located at the 3' end that serves to determine the site from which DNA polymerase starts amplification satisfies the aforementioned condition. Fixation of the base located at the 3' end of each primer in this manner makes it possible to sufficiently prevent the primer set (1) from being bound to, for example, another similar isozyme gene (for example, NAT1, NTA6, NAT8, or NAT9 gene).

As described above, since the F1 primer and R1 primer each can be any primer as long as the base located at the 3' end is fixed, the length itself of each primer is not particularly limited and can be adjusted suitably to be common length. The length of the primers is, for example, in the range of 13- to 50-mers, preferably 14- to 45-mers, and more preferably 15- to 40-mers. Specifically, it is preferable that the F1 primer be: at least one oligonucleotide having a sequence identical to that of a region extending from guanine (G) at base 1038 to be considered as the first base to any one of the 20^{th} to 32^{nd} bases (preferably the 24^{th} to 30^{th} bases and more preferably the 25^{th} to 29^{th} bases) in the direction toward the 5' end in the base sequence of SEQ ID NO: 1. Furthermore, it is preferable that the R1 primer be: at least one oligonucleotide complementary to a region extending from cytosine (C) at base 1096 to be considered as the first base to any one of the 17^{th} to 24^{th} bases (preferably the 19^{th} to 23^{rd} bases and more preferably the 20^{th} to 22^{nd} bases) in the direction toward the 3' end in the base sequence of SEQ ID NO: 1. Since the 3' end of the F1 primer and the R1 primer is fixed, the region to be elongated from the primer is, for example, one of a region from base 1039 to base 1095 in SEQ ID NO: 1 as described above. However, the length of the whole amplification product obtained varies according to the length of the primer to be used.

Furthermore, it is not necessary for the R1 primer and the F1 primer to be oligonucleotides perfectly complementary to the base sequence indicated in SEQ ID NO: 1 and to the strand complementary to the base sequence, respectively. In other words, the part excluding the base located at the 3' end in each primer may be different in one to five bases from that of a perfectly complementary oligonucleotide.

Specific examples of the F1 primer and the R1 primer are indicated below but the present invention is not limited thereto. The combination of these F1 primer and R1 primer is not limited by any means. Specifically, however, a primer set (1') is particularly preferable, which includes a F1' primer composed of oligonucleotide of SEQ ID NO: 5 or SEQ ID NO: 7, and a R1' primer composed of oligonucleotide of SEQ ID NO: 16 or SEQ ID NO: 18. "Tm (°C)" indicated below in the table is Tm (°C) obtained when each sequence indicated below in the table was hybridized with the sequence perfectly complementary thereto. The "Tm (°C)" is a value calculated by using MELTCALC software (http://www.meltcalc.com/), with parameters including an oligonucleotide concentration of 0.2 µM and a sodium equivalent (Na eq.) of 50 mM (the same applies below). The Tm value can be calculated by using, for example, conventionally known MELTCALC software (http://www.meltcalc.com/) or also can be determined by the nearest neighbor method (the same applies below).

**[Table 1]**

| Primer | Sequence | Tm(°C) | SEQ ID NO. |
|---|---|---|---|
| F1 Primer for NAT2*5 | 5'-ccctccagttaacaaatacagcactggcatgg-3' | 64 | 2 |
| | 5'-cctccagttaacaaatacagcactggcatgg-3' | 62.7 | 3 |
| | 5'-ctccagttaacaaatacagcactggcatgg-3' | 61.4 | 4 |
| | 5'-tccagttaacaaatacagcactggcatgg-3' | 60.9 | 5 |
| | 5'-ccagttaacaaatacagcactggcatgg-3' | 60.1 | 6 |
| | 5'-cagttaacaaatacagcactggcatgg-3' | 58.6 | 7 |
| | 5'-agttaacaaatacagcactggcatgg-3' | 57.7 | 8 |
| | 5'-gttaacaaatacagcactggcatgg-3' | 56.8 | 9 |
| | 5'-ttaacaaatacagcactggcatgg-3' | 55.8 | 10 |
| | 5'-taacaaatacagcactggcatgg-3' | 55.3 | 11 |
| | 5'-aacaaatacagcactggcatgg-3' | 55.6 | 12 |
| | 5'-acaaatacagcactggcatgg-3' | 55.2 | 13 |
| | 5'-caaatacagcactggcatgg-3' | 53.6 | 14 |
| R1 Primer for NAT2*5 | 5'-gccacatctgggaggagcttccag-3' | 62.5 | 15 |
| | 5'-ccacatctgggaggagcttccag-3' | 60.1 | 16 |
| | 5'-cacatctgggaggagcttccag-3' | 58.2 | 17 |
| | 5'-acatctgggaggagcttccag-3' | 57.1 | 18 |
| | 5'-catctgggaggagcttccag-3' | 55.6 | 19 |
| | 5'-atctgggaggagcttccag-3' | 54.2 | 20 |
| | 5'-tctgggaggagcttccag-3' | 53.8 | 21 |
| | 5'-ctgggaggagcttccag-3' | 52.2 | 22 |

Next, as described above, the primer set (2) is a primer set of a pair of primers including a forward primer composed of the following oligonucleotide (F2) and a reverse primer composed of the following oligonucleotide (R2).
(F2): at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
(R2): at least one oligonucleotide selected from:
   at least one oligonucleotide complementary to a region extending from guanine (G) at base 1355 to be considered as the first base to any one of the 25^{th} to 40^{th} bases in the direction toward the 3' end in the base sequence of SEQ ID NO: 1, with cytosine (C) complementary to the guanine (G) at base 1355 being the 3' end, and
   at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end.

The primer set (2) is a primer set for amplifying a DNA strand including a region from base 1279 to base 1354 or base 1279 to base 1613 in SEQ ID NO: 1 as well as a strand complementary thereto. Base 1312 in this region (i.e. base 1312 in SEQ ID NO: 1) is known for the presence of a point mutation (1312G, 1312A) that affects the function of NAT2, and the polymorphism thereof is NAT2*6 described above. In the present invention, the polymorphism of this site can be indicated as 1312G/G or 1312A/A in the case of homozygote and as 1312G/Ain the case of heterozygote. Further, since base 1312 in SEQ ID NO: 1 corresponds to base 590 in mRNA of the NAT2 gene, as the polymorphism NAT2*6, it also can be indicated, for example, as 590G/G, 590A/A, or 590G/A. Hereinafter, this primer set (2) also may be referred to as a "primer set for NAT2*6". When only the polymorphism NAT2*6 is to be analyzed, it is sufficient to use only the primer set for NAT2*6.

From the same reason as that described with respect to the primer set (1), in the present invention, the F2 primer and the R2 primer of the primer set (2) can be any primers, as long as the base located at the 3' end that serves to determine the site from which DNA polymerase starts amplification satisfies the aforementioned condition. Accordingly, the length itself of the F2 primer and the R2 primer is not particularly limited and can be, for example, as described above. Specifically, it is preferable that the F2 primer be at least one oligonucleotide having a sequence identical to that of a region extending from cytosine (C) at base 1278 to be considered as the first base to any one of the 20^{th} to 38^{th} bases (preferably the 21^{st} to 38^{th} bases and more preferably the 22^{nd} to 38^{th} bases) in the direction toward the 5' end in the base sequence of SEQ ID NO: 1. Furthermore, it is preferable that the R2 primer be: at least one oligonucleotide complementary to a region extending from guanine (G) at base 1355 to be considered as the first base to any one of the 25^{th} to 40^{th} bases (preferably the 27^{th} to 35^{th} bases and more preferably the 28^{th} to 34^{th} bases) in the direction toward the 3' end in the base sequence of SEQ ID NO: 1; or at least one oligonucleotide complementary to a region extending from cytosine (C) at base 1614 to be considered as the first base to any one of the 21^{st} to 36^{th} bases (preferably the 23^{rd} to 36^{th} bases and more preferably the 24^{th} to 36^{th} bases) in the direction toward the 3' end in the base sequence of SEQ ID NO: 1. Since each 3' end of the F2 primer and the R2 primer is fixed, the region to be elongated from the primer is, normally, a region from base 1279 to base 1354 or a region from base 1279 to base 1613 in SEQ ID NO: 1 as described above. However, the length of the whole amplification product obtained varies according to the length of the primer to be used.

Furthermore, it is not necessary for the R2 primer and the F2 primer to be oligonucleotides perfectly complementary to the base sequence indicated in SEQ ID NO: 1 and to the strand complementary to the base sequence, respectively. In other words, the part excluding the base located at the 3' end in each primer may be different in one to five bases from that of a perfectly complementary oligonucleotide.

Specific examples of the F2 primer and the R2 primer are indicated below but the present invention is not limited thereto. The combination of these F2 primer and R2 primer is not limited by any means. Specifically, however, a primer set (2') is particularly preferable, which includes a F2' primer composed of oligonucleotide of SEQ ID NO: 33 or SEQ ID NO: 109, and a R2' primer composed of oligonucleotide of SEQ ID NO: 39 or SEQ ID NO: 48.

**[Table 2]**

| Primer | Sequence | Tm(°C) | SEQ ID NO. |
|---|---|---|---|
| F2 Primer for NAT2*6 and NAT2*7 | 5'-agaatttcttaattctcatctcctgccaaagaagaaac-3' | 60.5 | 109 |
| | 5'-gaatttcttaattctcatctcctgccaaagaagaaac-3' | 59.8 | 110 |
| | 5'-aatttcttaattctcatctcctgccaaagaagaaac-3' | 59.4 | 111 |
| | 5'-atttcttaattctcatctcctgccaaagaagaaac-3' | 59.2 | 112 |
| | 5'-tttcttaattctcatctcctgccaaagaagaaac-3' | 59.2 | 23 |
| | 5'-ttcttaattctcatctcctgccaaagaagaaac-3' | 59 | 24 |
| | 5'-tcttaattctcatctcctgccaaagaagaaac-3' | 58.8 | 25 |
| | 5'-cttaattctcatctcctgccaaagaagaaac-3' | 58 | 26 |
| | 5'-ttaattctcatctcctgccaaagaagaaac-3' | 57.4 | 27 |
| | 5'-taattctcatctcctgccaaagaagaaac-3' | 57.1 | 28 |
| | 5'-aattctcatctcctgccaaagaagaaac-3' | 57.4 | 29 |
| | 5'-attctcatctcctgccaaagaagaaac-3' | 57.1 | 30 |
| | 5'-ttctcatctcctgccaaagaagaaac-3' | 57 | 31 |
| | 5'-tctcatctcctgccaaagaagaaac-3' | 56.6 | 32 |
| | 5'-ctcatctcctgccaaagaagaaac-3' | 55.6 | 33 |
| | 5'-tcatctcctgccaaagaagaaac-3' | 54.7 | 34 |
| | 5'-catctcctgccaaagaagaaac-3' | 53.5 | 35 |
| | 5'-atctcctgccaaagaagaaac-3' | 52.1 | 36 |
| | 5'-tctcctgccaaagaagaaac-3' | 51.7 | 37 |
| R2 Primer for NAT2*6 | 5'-ggaacaaaatgatgtggttataaatgaagatgttggagac-3' | 61.1 | 38 |
| | 5'-gaacaaaatgatgtggttataaatgaagatgttggagac-3' | 60 | 39 |
| | 5'-aacaaaatgatgtggttataaatgaagatgttggagac-3' | 59.7 | 40 |
| | 5'-acaaaatgatgtggttataaatgaagatgttggagac-3' | 59.5 | 41 |
| | 5'-caaaatgatgtggttataaatgaagatgttggagac-3' | 58.7 | 42 |
| | 5'-aaaatgatgtggttataaatgaagatgttggagac-3' | 58 | 43 |
| | 5'-aaatgatgtggttataaatgaagatgttggagac-3' | 57.8 | 44 |
| | 5'-aatgatgtggttataaatgaagatgttggagac-3' | 57.6 | 45 |
| | 5'-atgatgtggttataaatgaagatgttggagac-3' | 57.3 | 46 |
| | 5'-tgatgtggttataaatgaagatgttggagac-3' | 57.2 | 47 |
| | 5'-gatgtggttataaatgaagatgttggagac-3' | 56.2 | 48 |
| | 5'-atgtggttataaatgaagatgttggagac-3' | 55.5 | 49 |
| | 5'-tgtggttataaatgaagatgttggagac-3' | 55.3 | 50 |
| | 5'-gtggttataaatgaagatgttggagac-3' | 54.1 | 51 |
| | 5'-tggttataaatgaagatgttggagac-3' | 53 | 52 |
| | 5'-ggttataaatgaagatgttggagac-3' | 51.7 | 53 |

Next, as described above, the primer set (3) is a primer set of a pair of primers including a forward primer composed of the following oligonucleotide (F3) and a reverse primer composed of the following oligonucleotide (R3). (F3): at least one oligonucleotide selected from:
at least one oligonucleotide having a sequence identical to that of a region extending from thymine (T) at base 1556 to be considered as the first base to any one of the 21^{st} to 40^{th} bases in the direction toward the 5' end in the base sequence of SEQ ID NO: 1, with the thymine (T) being the 3' end, and
at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
(R3): at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end.

The primer set (3) is a primer set for amplifying a DNA strand including a region from base 1557 to base 1613 or a region from base 1279 to base 1613 in SEQ ID NO: 1 as well as a strand complementary thereto. Base 1579 in this region (i.e. base 1579 in SEQ ID NO: 1) is known for the presence of a point mutation (1579G, 1579A) that affects the function of NAT2, and the polymorphism thereof is NAT2*7 described above. In the present invention, the polymorphism of this site can be indicated as 1579G/G or 1579A/A in the case of homozygote and as 1579G/A in the case of heterozygote. Further, since base 1579 in SEQ ID NO: 1 corresponds to base 857 in mRNA of the NAT2 gene, as the polymorphism NAT2*7, it can also be indicated, for example, as 857G/G, 857A/A, or 857G/A. Hereinafter, this primer set (3) also may be referred to as a "primer set for NAT2*7". When only the polymorphism NAT2*7 is to be analyzed, it is sufficient to use only the primer set for NAT2*7.

In the present invention, for the same reason as that described with respect to the primer set (1), the F3 primer and the R3 primer of the primer set (3) can be any primers as long as the base located at the 3' end that serves to determine the site from which DNA polymerase starts amplification satisfies the aforementioned condition. Accordingly, the length itself of the F3 primer and the R3 primer is not particularly limited and can be, for example, as described above. Specifically, it is preferable that the F3 primer be: at least one oligonucleotide having a sequence identical to that of a region extending from thymine (T) at base 1556 to be considered as the first base to any one of the 21^{st} to 40^{th} bases (preferably the 23^{rd} to 40^{th} bases and more preferably the 24^{th} to 40^{th} bases) in the direction toward the 5' end in the base sequence of SEQ ID NO: 1; or at least one oligonucleotide having a sequence identical to that of a region extending from cytosine (C) at base 1278 to be considered as the first base to any one of the 20^{th} to 38^{th} bases (preferably the 21^{st} to 38^{th} bases and more preferably the 22^{nd} to 38^{th} bases) in the direction toward the 5' end in the base sequence of SEQ ID NO: 1. Furthermore, it is preferable that the R3 primer be at least one oligonucleotide complementary to a region extending from cytosine (C) at base 1614 to be considered as the first base to any one of the 21^{st} to 36^{th} bases (preferably the 23^{rd} to 35^{th} bases and more preferably the 24^{th} to 28^{th} bases) in the direction toward the 3' end in the base sequence of SEQ ID NO: 1. Since each 3' end of the F3 primer and the R3 primer is fixed, the region to be elongated from the primer is, for example, a region from base 1557 to base 1613 or a region from base 1279 to base 1613 in SEQ ID NO: 1 as described above. However, the length of the whole amplification product obtained varies according to the length of the primer to be used.

Furthermore, it is not necessary for the R3 primer and the F3 primer to be oligonucleotides perfectly complementary to the base sequence indicated in SEQ ID NO: 1 and to the strand complementary to the base sequence, respectively. In other words, the part excluding the base located at the 3' end in each primer may be different in one to five bases from that of a perfectly complementary oligonucleotide.

Specific examples of the F3 primer and the R3 primer are indicated below but the present invention is not limited thereto. The combination of these F3 primer and R3 primer is not limited by any means. Specifically, however, a primer set (3') is particularly preferable, which includes a F3' primer composed of oligonucleotide of SEQ ID NO: 60 or SEQ ID NO: 113 and a R3' primer composed of oligonucleotide of SEQ ID NO: 71 or SEQ ID NO: 81.

**[Table 3]**

| Primer | Sequence | Tm(°C) | SEQ ID NO. |
|---|---|---|---|
| F3 Primer for NAT2*7 | 5'-agtgctgagaaatatatttaagatttccttggggagaaat-3' | 60.7 | 113 |
| | 5'-gtgctgagaaatatatttaagatttccttggggagaaat-3' | 60.1 | 114 |
| | 5'-tgctgagaaatatatttaagatttccttggggagaaat-3' | 59.5 | 115 |
| | 5'-gctgagaaatatatttaagatttccttggggagaaat-3' | 58.7 | 54 |
| | 5'-ctgagaaatatatttaagatttccttggggagaaat-3' | 57.1 | 55 |
| | 5'-tgagaaatatatttaagatttccttggggagaaat-3' | 56.6 | 56 |
| | 5'-gagaaatatatttaagatttccttggggagaaat-3' | 55.6 | 57 |
| | 5'-agaaatatatttaagatttccttggggagaaat-3' | 55 | 58 |
| | 5'-gaaatatatttaagatttccttggggagaaat-3' | 54.2 | 59 |
| | 5'-aaatatatttaagatttccttggggagaaat-3' | 53.4 | 60 |
| | 5'-aatatatttaagatttccttggggagaaat-3' | 53 | 61 |
| | 5'-atatatttaagatttccttggggagaaat-3' | 52.5 | 62 |
| | 5'-tatatttaagatttccttggggagaaat-3' | 52.2 | 63 |
| | 5'-atatttaagatttccttggggagaaat-3' | 52.4 | 64 |
| | 5'-tatttaagatttccttggggagaaat-3' | 52 | 65 |
| | 5'-atttaagatttccttggggagaaat-3' | 52.2 | 66 |
| | 5'-tttaagatttccttggggagaaat-3' | 51.8 | 67 |
| | 5'-ttaagatttccttggggagaaat-3' | 51.2 | 68 |
| | 5'-taagatttccttggggagaaat-3' | 50.5 | 69 |
| | 5'-aagatttccttggggagaaat-3' | 50.6 | 70 |
| R3 Primer for NAT2*7 | 5'-tgataattagtgagttgggtgatacatacacaaggg-3' | 60.7 | 71 |
| | 5'-gataattagtgagttgggtgatacatacacaaggg-3' | 59.9 | 72 |
| | 5'-ataattagtgagttgggtgatacatacacaaggg-3' | 59.5 | 73 |
| | 5'-taattagtgagttgggtgatacatacacaaggg-3' | 59.4 | 74 |
| | 5'-aattagtgagttgggtgatacatacacaaggg-3' | 59.7 | 75 |
| | 5'-attagtgagttgggtgatacatacacaaggg-3' | 59.5 | 76 |
| | 5'-ttagtgagttgggtgatacatacacaaggg-3' | 59.5 | 77 |
| | 5'-tagtgagttgggtgatacatacacaaggg-3' | 59.3 | 78 |
| | 5'-agtgagttgggtgatacatacacaaggg-3' | 59.6 | 79 |
| | 5'-gtgagttgggtgatacatacacaaggg-3' | 58.8 | 80 |
| | 5'-tgagttgggtgatacatacacaaggg-3' | 57.9 | 81 |
| | 5'-gagttgggtgatacatacacaaggg-3' | 56.7 | 82 |
| | 5'-agttgggtgatacatacacaaggg-3' | 55.9 | 83 |
| | 5'-gttgggtgatacatacacaaggg-3' | 54.8 | 84 |
| | 5'-ttgggtgatacatacacaaggg-3' | 53.6 | 85 |
| | 5'-tgggtgatacatacacaaggg-3' | 53 | 86 |

Furthermore, the aforementioned each primer may be, for example, one with the 5' end to which any conventionally known sequence has been added in order to increase the amplification reaction temperature.

In a case where a DNA strand including a region from base 1279 to base 1613 as well as a strand complementary thereto is amplified by the aforementioned primer set (2) or the aforementioned primer set (3), the same forward primer and reverse primer indicated as follows can be used for either primer set:
forward primer:
   (F2) (F3): at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and reverse primer
   (R2) (R3): at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end.

In this case, besides the primer set (1), the primer set of the present invention may include only, for example, one type of primer set (primer for NAT2*6*7) as the primer set (2) and the primer set (3). Further, as for the forward primer, the primer set of the present invention may include one type of forward primer that serves as the forward primer for both of the primer set (2) and the primer set (3), and as for the reverse primer, the primer set of the present invention may include respective reverse primers for each of the primer set (2) and the primer set (3).

Preferably, a primer set for amplifying the NAT2 gene of the present invention including at least one of the aforementioned primer sets (1) to (3) is used, for example, in amplifying the NAT2 gene in a biological sample such as a whole blood sample. Particularly, when the primer set for amplifying the NAT2 gene of the present invention is used in combination with a probe for detecting a polymorphism as described later, it is preferable that the ratio of the whole blood sample to be added to the reaction solution for amplifying a gene be 0.1 to 0.5 vol%. This will be described later.

### < Reagent for amplifying NAT2 gene>

As described above, a reagent for amplifying the NAT2 gene of the present invention is a reagent for amplifying the NAT2 gene by a gene amplification method, wherein the reagent includes a primer set for amplifying the NAT2 gene of the present invention. The reagent for amplifying the NAT2 gene of the present invention is characterized by including a primer set of the present invention and, for example, compositions of other than this are not limited by any means.

For example, in order to detect an amplification product obtained by a gene amplification method in which a primer set of the present invention is used, the reagent for amplifying the NAT2 gene of the present invention further may include a probe that can hybridize to a site to be detected in the NAT2 gene. As described above, the primer set of the present invention allows specific amplification of one to three target regions in the NAT2 gene by a gene amplification method according to, for example, the type of the primer sets (1) to (3) included therein. Accordingly, when a probe complementary to the sequence to be detected in each target region described above is allowed to coexist, for example, the presence or absence of amplification or the genotype (polymorphism) of the site to be detected can be detected by the method described later. Such probes and the method of using them are explained later in the description of the polymorphism analysis method. Furthermore, it is preferable that the reagent for amplifying the NAT2 gene of the present invention be used in amplifying the NAT2 gene in a biological sample such as whole blood. Particularly, when the reagent for amplifying the NAT2 gene of the present invention is used in combination with the probe described above, it is preferable that the ratio of the whole blood sample to be added to the reaction solution for amplifying a gene be 0.1 to 0.5 vol%. In the present invention, the term "sequence to be detected" denotes a sequence including a site (site to be detected) at which a polymorphism is generated.

The form of the reagent for amplifying the NAT2 gene of the present invention is not particularly limited and it may be, for example, a liquid reagent containing a primer set for amplifying the NAT2 gene of the present invention or a dry reagent that is to be suspended in a solvent before use. Furthermore, the content of the primer set for amplifying the NAT2 gene also is not particularly limited.

### <Method of manufacturing amplification product>

As described above, the method of manufacturing an amplification product of the present invention is a method of manufacturing an amplification product of the NAT2 gene by a gene amplification method, wherein the method includes the following step (I):
(I) amplifying the NAT2 gene in a reaction solution using a primer set for amplifying the NAT2 gene of the present invention, with nucleic acid contained in a sample being used as a template.

When a primer set for amplifying the NAT2 gene of the present invention is used to perform an amplification reaction in this manner, the target region of the NAT2 gene can be amplified as described above. Furthermore, when the primer set for amplifying the NAT2 gene of the present invention includes two of the primer sets (1) to (3), two target regions including two sites to be detected, respectively, in the NAT2 gene can be amplified simultaneously in the same reaction solution. Moreover, when the primer set for amplifying the NAT2 gene of the present invention includes all the primer sets (1) to (3), three target regions including three sites to be detected, respectively, in the NAT2 gene can be amplified simultaneously in the same reaction solution. The target regions to be amplified according to the present invention are regions including the sites to be detected at which respective polymorphisms (NAT2*5, NAT2*6, and NAT2*7) are generated, respectively, as described above. The method of manufacturing an amplification product of the present invention is **characterized in that** a primer set of the present invention is used, and, for example, the type of and conditions for the gene amplification method are not limited by any means.

The gene amplification method is not particularly limited as described above. Examples thereof include the polymerase chain reaction (PCR) method, a nucleic acid sequence based amplification (NASBA) method, a transcription-mediated amplification (TMA) method, and a strand displacement amplification (SDA) method. However, the PCR method is preferable. The present invention is described below using the PCR method as an example but is not limited thereby.

The sample to which the present invention is to be applied is not particularly limited as long as it contains, for example, nucleic acid to serve as a template. However, it is preferable that the present invention be applied to, for example, a contaminated sample. Examples of the contaminated sample include whole blood, cells in the mouth (for example, oral mucosa), somatic cells of nails and hairs, germ cells, expectoration, amniotic fluid, paraffin-embedded tissue, urine, gastric juice (for example, gastric lavage fluid), and suspensions thereof. According to the method of manufacturing an amplification product using a primer set of the present invention, for example, even in the case of a sample (particularly, a biological sample such as whole blood or cells in the mouth) with various contaminants, the method is less subject to the effect thereof and allows the target region in the NAT2 gene to be amplified specifically. Thus, according to the present invention, even a highly contaminated sample, which is difficult to use in the conventional methods, can be used as it is, for instance, without being pretreated, for example, without being purified. Therefore, it can be said that an amplification product can be prepared quicker as compared to the conventional method also from the viewpoint of the pretreatment of the sample.

The ratio of the sample to be added to the reaction solution is not particularly limited. Specifically, when the sample is a biological sample (for example, a whole blood sample), the lower limit of the ratio thereof to be added to the reaction solution is, for example, preferably at least 0.01 vol%, more preferably at least 0.05 vol%, and further preferably at least 0.1 vol%. Furthermore, the upper limit of the ratio thereof to be added also is not particularly limited and is, for example, preferably 2 vol% or lower, more preferably 1 vol% or lower, and further preferably 0.5 vol% or lower.

When an optical detection to be described later is intended to be performed, particularly, when an optical detection is performed using a labeled probe, it is preferable that the ratio of a biological sample, such as a whole blood sample, to be added to the reaction solution be set at, for example, 0.1 to 0.5 vol%. Generally, in the PCR reaction, a heat treatment is carried out to denature DNA (i.e. to dissociate it into a single-stranded DNA). This heat treatment may denature, for example, sugar or protein contained in the sample and thereby may generate an insolubilized precipitate or turbidity. Therefore, when the presence or absence of an amplification product or the genotype (polymorphism) of a site to be detected is to be checked by an optical method, the generation of such a precipitate or turbidity may affect measurement accuracy. However, when the ratio of the whole blood sample to be added to the reaction solution is set in the range described above, for example, an effect caused by generation of, for example, a precipitate due to denaturation can be prevented sufficiently and thereby the accuracy of measurement carried out by the optical method can be improved, although the mechanism thereof is unknown. Furthermore, since it also sufficiently can prevent PCR from being inhibited due to the contaminants contained in a whole blood sample, the amplification efficiency can be improved further. Accordingly, when in addition to the use of a primer set of the present invention, the ratio of the sample such as a whole blood sample to be added is set in the aforementioned range, further the need to pretreat the sample can be omitted.

Furthermore, the ratio of the whole blood sample in the reaction solution can be indicated not in the aforementioned volume ratio (for example, 0.1 to 0.5 vol%) but in a weight ratio of hemoglobin (hereinafter referred to as "Hb"). In this case, the ratio of the whole blood sample in the reaction solution is, for example, preferably in the range of 0.565 to 113 g/L, more preferably in the range of 2.825 to 56.5 g/L, and further preferably in the range of 5.65 to 28.25 g/L, in terms of the amount of Hb. The ratio of the whole blood sample to be added during the reaction may satisfy, for example, both the volume ratio and the Hb weight ratio, or one of them.

The whole blood may be any one of, for example, hemolyzed whole blood, unhemolyzed whole blood, anticoagulated whole blood, and whole blood containing coagulated fractions.

In the present invention, the target nucleic acid contained in a sample is, for example, DNA. The aforementioned DNA may be DNA contained originally in the sample, such as a biological sample, or an amplification product DNA obtained through amplification by a gene amplification method. In the latter case, an example thereof is cDNA that is generated from RNA (for example, total RNA or mRNA) contained originally in the sample by a reverse transcription reaction (for instance, reverse transcription PCR (RT-PCR)).

In the method of manufacturing an amplification product of the present invention, it is preferable that albumin further be added to the reaction solution before the start of a gene amplification reaction. Such addition of albumin further can reduce the effect of generation of a precipitate or turbidity described above and also further can improve the amplification efficiency. Specifically, it is preferable that albumin be added before the amplification reaction in step (I) or a step of dissociation into a single-stranded DNA.

The ratio of albumin to be added to the reaction solution is, for example, in the range of 0.01 to 2 wt%, preferably 0.1 to 1 wt%, and more preferably 0.2 to 0.8 wt%. The albumin is not particularly limited. Examples thereof include bovine serum albumin (BSA), human serum albumin, rat serum albumin, and horse serum albumin. One of them may be used or two or more of them may be used in combination.

Next, a method of manufacturing an amplification product of the present invention is described using an example in which, with respect to a whole blood sample including DNA as target nucleic acid, amplification products of three target regions of the NAT2 gene are produced by PCR using primer sets for amplifying the NAT2 gene of the present invention including the aforementioned primer sets (1) to (3). The present invention is characterized by using primer sets of the present invention and other configurations and conditions are not limited by any means.

First, a PCR reaction solution is prepared. The ratio of the primer sets of the present invention to be added is not particularly limited. However, it is preferable that F primers of the primer sets (1) to (3) each be added to be 0.1 to 2 µmol/L, more preferably 0.25 to 1.5 µmol/L, and particularly preferably 0.5 to 1 µmol/L. Furthermore, it is preferable that R primers of the primer sets (1) to (3) each be added to be 0.1 to 2 µmol/L, more preferably 0.25 to 1.5 µmol/L, and particularly preferably 0.5 to 1 µmol/L. The ratio (F:R, molar ratio) between the F primer and the R primer to be added to each primer set is not particularly limited. It is, for example, preferably 1 : 0.25 to 1 : 4 and more preferably 1 : 0.5 to 1 : 2.

The ratio of the whole blood sample in the reaction solution is not particularly limited but is preferably in the range described above. The whole blood sample may be added to the reaction solution without being treated or may be added to the reaction solution after being diluted with a solvent such as water or a buffer solution beforehand. When the whole blood sample is diluted beforehand, the dilution ratio is not particularly limited. It can be set so that, for example, the final ratio of the whole blood added to the reaction solution is in the aforementioned range, for example, 1:100 to 1:2000 and preferably 1:200 to 1:1000.

Other composition components in the reaction solution are not particularly limited and can be conventionally known components, whose ratios also are not particularly limited. Examples of the composition components include DNA polymerase, nucleotide (nucleoside triphosphate (dNTP)), and a solvent. Furthermore, as described above, it is preferable that the reaction solution further contain albumin. In the reaction solution, the order of addition of the respective composition components is not limited by any means.

The DNA polymerase is not particularly limited and, for example, a conventionally known thermoduric bacteria-derived polymerase can be used. Specifically, for example, Thermus aquaticus-derived DNA polymerase (USP 4,889,818 and USP 5,079,352) (trade name: Taq polymerase), Thermus thermophilus-derived DNA polymerase (WO 91/09950) (rTth DNA polymerase), Pyrococcus furiosus-derived DNA polymerase (WO 92/9689) (Pfu DNA polymerase; manufactured by Stratagenes), and Thermococcus litoralis-derived DNA polymerase (EP-A 455 430) (Trademark: Vent; manufactured by New England Biolabs) are commercially available. Particularly, Thermus aquaticus-derived thermostable DNA polymerase is preferable.

The ratio of DNA polymerase to be added to the reaction solution is not particularly limited and is, for example, 1 to 100 U/mL, preferably 5 to 50 U/mL, and more preferably 20 to 30 U/mL. With respect to the unit of activity (U) of DNA polymerase, generally, 1 U denotes the activity that allows all 10 nmol of nucleotide to be taken into an acid-insoluble precipitate in 30 minutes at 74°C in a reaction solution for activity measurement, with an activated salmon sperm DNA being used as a template primer. The composition of the reaction solution for activity measurement is, for example, 25 mM TAPS buffer (pH 9.3, 25°C), 50 mM KCl, 2 mM MgCl₂, 1 mM mercaptoethanol, 200 µM dATP, 200 µM dGTP, 200 µM dTTP, 100 µM [α-³²P] dCTP, and 0.25 mg/mL activated salmon sperm DNA.

Generally, examples of the nucleoside triphosphate include dNTP (dATP, dCTP, dTTP). The ratio of dNTP to be added to the reaction solution is not particularly limited and is, for example, 0.01 to 1 mmol/L, preferably 0.05 to 0.5 mmol/L, and more preferably 0.1 to 0.3 mmol/L.

Examples of the solvent include buffer solutions such as Tris-HCl, Tricine, MES, MOPS, HEPES, and CAPS. Commercially available PCR buffer solutions or buffer solutions of commercially available PCR kits can be used.

Furthermore, the PCR reaction solution further may contain heparin, betaine, KCl, MgCl₂, MgSO₄, glycerol, etc. The ratios thereof to be added can be set in ranges in which the PCR reaction is not inhibited.

The total volume of the reaction solution is not particularly limited and can be determined suitably according to, for example, the equipment (thermal cycler) to be used. It is generally 1 to 500 µL and preferably 10 to 100 µL.

Subsequently, PCR is performed. The cycle conditions in PCR are not particularly limited, and, for example, (1) dissociation of whole blood-derived double-stranded DNA into single-stranded DNA, (2) annealing of a primer, and (3) elongation of a primer (polymerase reaction) are as described below. Furthermore, the number of cycles also is not particularly limited but preferably is at least 30, with the following three steps (1) to (3) being considered as one cycle. The upper limit thereof, in total, is not particularly limited and, for example, 100 cycles or less, preferably 70 cycles or less, and further preferably 50 cycles or less. The change in temperature in each step can be controlled automatically using, for example, a thermal cycler. When primer sets of the present invention are used, since they are excellent in amplification efficiency as described above, 50 cycles can be completed in approximately one hour (preferably within one hour) according to the present invention, while it takes approximately three hours to complete 50 cycles according to the conventional methods.

**[Table 4]**

| | Temperature (°C) and Time (sec) |
|---|---|
| (1) Dissociation of single-stranded DNA | For example, 90 to 99°C, 1 to 120 sec |
| | Preferably, 92 to 95°C, 1 to 60 sec |
| (2) Annealing of primer | For example, 40 to 70°C, 1 to 300 sec |
| | Preferably, 50 to 70°C, 5 to 60 sec |
| (3) Elongation reaction | For example, 50 to 80°C, 1 to 300 sec |
| | Preferably, 50 to 75°C, 5 to 60 sec |

In the manner described above, amplification products complementary to the three target regions in the NAT2 gene can be produced. When an amplification product complementary to one or those complementary to two of the three target regions are to be produced, a primer set for amplifying the NAT2 gene of the present invention containing one or two of the primer sets (1) to (3) corresponding to the target region(s) can be used.

The method of manufacturing an amplification product of the present invention further may include a step of detecting an amplification product of a region obtained by the aforementioned amplification reaction. This makes it possible to detect the presence or absence of the amplification product or the genotype (polymorphism, NAT2*5, NAT2*6, or NAT2*7) in the target region in the NAT2 gene. The presence or absence of the amplification product can be checked by a conventionally known method. Specifically, it can be checked by, for example, further adding a probe (for instance, a fluorescently-labeled probe) that can hybridize to a site to be detected in the NAT2 gene to the reaction solution in step (I), and further in step (II), measuring the fluorescence intensity of the fluorescent label in the probe with respect to the reaction solution. Furthermore, when two or three target regions are to be amplified, it can be checked by, for example, further adding two or three probes (for instance, fluorescently-labeled probes) that can hybridize to the respective sites to be detected in the NAT2 gene to the reaction solution in step (I), and further in step (II), measuring the fluorescence intensity of the fluorescent label in each probe with respect to the reaction solution. Detection of polymorphisms, NAT2*5, NAT2*6, and NAT2*7, in the NAT2 gene is described below as an embodiment of the present invention.

### < NAT2 gene polymorphism analysis method>

NAT2 gene polymorphism analysis method of the present invention is a method of analyzing the polymorphism of a site to be detected in the NAT2 gene, wherein the method includes the following steps (i) to (iv):
(i) amplifying a region including a site to be detected in the NAT2 gene in a reaction solution by a method of manufacturing an amplification product according to the present invention,
(ii) preparing a reaction solution that contains the amplification product obtained in step (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

In this manner, when an amplification product is produced using a primer set of the present invention, it is possible to amplify the target region including a site to be detected of a polymorphism (NAT2*5, NAT2*6, or NAT2*7) in the NAT2 gene as described above and to analyze the polymorphism of the site to be detected in the target region.

The probe to be used in step (i) is not particularly limited. Examples thereof include a probe that hybridizes to the site where the polymorphism NAT2*5 is generated (hereinafter, also referred to as a "probe for NAT2*5"), a probe that hybridizes to the site where the polymorphism NAT2*6 is generated (hereinafter, also referred to as a "probe for NAT2*6"), and a probe that hybridizes to the site where the polymorphism NAT2*7 is generated (hereinafter, also referred to as a "probe for NAT2*7"). Preferably, these probes each are a probe complementary to a sequence to be detected containing the aforementioned site to be detected. Any one of those probes may be used or two or all three of them may be used. This can be determined, for example, according to the type of the target region(s) amplified with a primer set for amplifying the NAT2 gene of the present invention. When two or three probes are used, for example, the polymorphisms of two sites to be detected or all the three sites to be detected can be analyzed using the same reaction solution.

The probes for detecting the polymorphisms are not particularly limited and can be configured by a conventionally known method. For instance, they each may be designed as a sequence to be detected containing a site to be detected of a polymorphism, based on the sequence of a sense strand or the sequence of an antisense strand of the NAT2 gene. Furthermore, the base located at the site of a polymorphism to be detected can be determined suitably according to the type of each polymorphism. In other words, in the case of NAT2*5, since the polymorphisms of "T" and "C" at base 1063 in SEQ ID NO: 1 have been known, examples of the probe include a probe complementary to either a sequence to be detected including T at base 1063 or a sequence to be detected including C at base 1063 (a probe for detecting a sense strand), and a probe complementary to a sequence of an antisense strand thereof (a probe for detecting an antisense strand). Furthermore, in the case of NAT2*6, since the polymorphisms of "G" and "A" at base 1312 in SEQ ID NO: 1 have been known, examples of the probe include a probe complementary to either a sequence to be detected including G at base 1312 or a sequence to be detected including A at base 1312 (a probe for detecting a sense strand), and a probe complementary to a sequence of an antisense strand thereof (a probe for detecting an antisense strand). Moreover, in the case of NAT2*7, since the polymorphisms of "G" and "A" at base 1579 in SEQ ID NO: 1 have been known, examples of the probe include a probe complementary to either a sequence to be detected including G at base 1579 or a sequence to be detected including A at base 1579 (a probe for detecting a sense strand), and a probe complementary to a sequence of an antisense strand thereof (a probe for detecting an antisense strand). As described above, when a probe is designed, with the base located at the site to be detected where a polymorphism is generated being set to be any one of the bases as described above, it is also possible to judge what type of polymorphism is expressed at each site to be detected in an NAT2 gene by the method as described later.

The probe can be added to an amplified reaction solution after step (i) i.e. after a target region in the NAT2 gene is subjected to an amplification reaction. However, it is preferable that the probe be added to a reaction solution before the amplification reaction in step (i) since this allows analysis to be performed easily and quickly.

The ratio of the probe to be added to the reaction solution is not particularly limited. For example, each probe is added to be preferably in the range of 10 to 400 nmol/L and more preferably in the range of 20 to 200 nmol/L. When a fluorescent dye is used as the label for a probe, an unlabeled probe with a sequence identical to that of the labeled probe may be used in combination with the labeled probe, for example, in order to adjust the fluorescence intensity to be detected, and the unlabeled probe may include phosphate group added to the 3' end thereof. In this case, the molar ratio between the labeled probe and the unlabeled probe is preferably, for example, 1 : 10 to 10 : 1. The length of the probe is not particularly limited. It is, for example, 5- to 50-mers and preferably 10- to 30-mers.

The Tm value is described. When a solution containing double-stranded DNA is heated, the absorbance at 260 nm increases. This is because heating releases the hydrogen bonds between both strands in the double-stranded DNA to dissociate it into single-stranded DNA (i.e. DNA melting). When all double-stranded DNAs are dissociated into single-stranded DNAs, the absorbance thereof indicates approximately 1.5 times that obtained at the start of heating (i.e. absorbance of only double-stranded DNAs), which makes it possible to judge that melting is completed thereby. Based on this phenomenon, the melting temperature Tm generally is defined as a temperature at which the absorbance has reached 50% of the total increase in absorbance.

In the aforementioned step (iii), the measurement of the signal values that indicate the melting states of the hybridization product between the amplification product and the probe may be a measurement of absorbance at 260 nm as described above but may be a measurement of the signal of a labeling substance. Specifically, it is preferable that a labeled probe labeled with a labeling substance be used as the aforementioned probe to perform the measurement of the signal of the labeling substance. The labeled probe can be, for example, a labeled probe that exhibits a signal independently but does not exhibit a signal after hybridization, or a labeled probe that does not exhibit a signal independently but exhibits a signal after hybridization. The former probe does not exhibit a signal after forming a hybrid (double-stranded DNA) with a sequence to be detected but exhibits a signal when the probe is released by heating. On the other hand, the latter probe exhibits a signal after forming a hybrid (double-stranded DNA) with a sequence to be detected but the signal is reduced (quenched) when the probe is released by heating. Accordingly, when the signal exhibited by the label is detected under a condition (absorption wavelength etc.) specific to the signal, the progress of melting of the hybridization product and the Tm value can be determined as in the case of the measurement of absorbance at 260 nm.

In the present invention, as described above, it is also possible to check polymorphisms with respect to amplification products of two or three target regions amplified in the same reaction solution. Accordingly, when two or three types of probes are used, it is preferable that they be labeled with different labels each of which is detected under its own condition. The use of different labels as described above makes it possible to analyze each amplification product separately by changing the detection conditions even in the same reaction solution.

Specific examples of labeling substances in the labeled probes include a fluorescent dye (fluorophore). A specific example of the labeled probes is preferably a probe that, for example, has been labeled with a fluorescent dye, exhibits fluorescence independently, and allows fluorescence to be reduced (for example, quenched) after hybridization. Generally, a probe that utilizes such a fluorescence quenching phenomenon is referred to as a fluorescence quenching probe. Particularly, with respect to the aforementioned probe, it is preferable that the 3' end or 5' end of oligonucleotide be labeled with a fluorescent dye and the base located at the end to be labeled be C. In this case, in the sequence to be detected, to which the labeled probe hybridizes, it is preferable that the base sequence of the labeled probe be designed so that the base to be paired with the end base C of the labeled probe or the base located 1 to 3 bases apart from the base to be paired be G. Generally, such a probe is referred to as a guanine quenching probe and is known as a so-called QProbe (registered trademark). When such a guanine quenching probe hybridizes to a sequence to be detected, C located at the end, which has been labeled with a fluorescent dye, approaches G in the DNA to be detected, and thereby a phenomenon occurs in which the emission of the fluorescent dye decreases (the fluorescence intensity decreases). The use of such a probe makes it possible to verify hybridization and dissociation easily according to a change in the signal.

The fluorescent dye is not particularly limited. Examples thereof include fluorescein, phosphor, rhodamine, and polymethine dye derivative. Examples of commercially available fluorescent dye include BODIPY FL (brand name, manufactured by Molecular Probe Inc.), FluorePrime (trade name, manufactured by Amersham Pharmacia), Fluoredite (trade name, manufactured by Millipore Corporation), FAM (manufactured by ABI), Cy3 and Cy5 (manufactured by Amersham Pharmacia), and TAMRA (manufactured by Molecular Probe Inc.). The combination of fluorescent dyes to be used for three types of probe is not particularly limited as long as, for example, it allows the respective probes to be detected under different conditions. Examples thereof include a combination of Pacific Blue (with a detection wavelength is 450 to 480 nm), TAMRA (with a detection wavelength is 585 to 700 nm), and BODIPY FL (with a detection wavelength is 515 to 555 nm).

Specific examples of the sequences of probes for detecting the polymorphisms, NAT2*5, NAT2 *6, and NAT2 *7, are indicated below, but the present invention is not limited thereto. The following probe (1) is an example of the probe for NAT2*5 and is a probe for detecting a sense strand. The following probe (2) is an example of the probe for NAT2*6, and is a probe for detecting an antisense strand. Furthermore, the following probe (3) is an example of the probe for NAT2*7 and is a probe for detecting an antisense strand.

### Probe (1)

(1-1) At least one oligonucleotide complementary to a region extending from guanine (G) at base 1056 to be considered as the first base to any one of the 13^{rd} to 19^{th} bases in the direction toward the 3' end in SEQ ID NO: 1, with cytosine complementary to the guanine (G) being the 3' end.

### Probe (2)

At least one oligonucleotide having a sequence identical to that of a region extending from cytosine (C) at base 1302 to be considered as the first base to any one of the 18^{th} to 27^{th} bases in the direction toward the 5' end in SEQ ID NO: 1, with the cytosine being the 5' end.

### Probe (3)

At least one oligonucleotide having a sequence identical to that of a region extending from cytosine (C) at base 1583 to be considered as the first base to any one of the 16^{th} to 21^{st} bases in the direction toward the 5' end in SEQ ID NO: 1, with the cytosine being the 3' end.

In the probe (1), base 1063 in SEQ ID NO: 1 is indicated with "r", and the "r" is A or G. In the probe (2), base 1312 in SEQ ID NO: 1 is indicated with "r", and the "r" is G or A. In the probe (3), base 1579 in SEQ ID NO: 1 is indicated with "r", and the "r" is G or A.

Specific examples of Probe (1), Probe (2), and Probe (3) are indicated in the following table. "Tm(°C)" indicated below in the table is Tm(°C) obtained when each sequence indicated below in the table was hybridized with the sequence perfectly complementary thereto. The "Tm(°C)" is a value calculated by using MELTCALC software (http://www.meltcalc.com/), with parameters including an oligonucleotide concentration of 0.2 µM and a sodium equivalent (Na eq.) of 50 mM.

**[Table 5]**

| Probe | Sequence | Tm(°C) | SEQ ID NO. |
|---|---|---|---|
| Probe (1) for NAT2*5 | 5'-tcctgccgtcaGtggtcac-3' | 58.1 | 87 |
| | 5'-cctgccgtcaGtggtcac-3' | 56.8 | 88 |
| | 5'-ctgccgtcaGtggtcac-3' | 54.2 | 89 |
| | 5'-tgccgtcaGtggtcac-3' | 52.9 | 90 |
| | 5'-gccgtcaGtggtcac-3' | 50.8 | 91 |
| | 5'-ccgtcaGtggtcac-3' | 46.3 | 92 |
| | 5'-cgtcaGtggtcac-3' | 42.3 | 116 |
| | 5'-tcctgccgtcaAtggtcac-3' | 56.2 | 117 |
| | 5'-cctgccgtcaAtggtcac-3' | 54.9 | 118 |
| | 5'-ctgccgtcaAtggtcac-3' | 52.2 | 119 |
| | 5'-tgccgtcaAtggtcac-3' | 50.7 | 120 |
| | 5'-gccgtcaAtggtcac-3' | 48.5 | 121 |
| | 5'-ccgtcaAtggtcac-3' | 43.8 | 122 |
| | 5'-cgtcaAtggtcac-3' | 39.6 | 123 |
| Probe (2) for NAT2*6 | 5'-cttgaacctcAaacaattgaagatttt-3' | 53.4 | 93 |
| | 5'-cttgaacctcAaacaattgaagattt-3' | 52.9 | 94 |
| | 5'-cttgaacctcAaacaattgaagatt-3' | 52.4 | 95 |
| | 5'-cttgaacctcAaacaattgaagat-3' | 51.8 | 96 |
| | 5'-cttgaacctcAaacaattgaaga-3' | 51.4 | 97 |
| | 5'-cttgaacctcAaacaattgaag-3' | 50.1 | 98 |
| | 5'-cttgaacctcAaacaattgaa-3' | 48.9 | 99 |
| | 5'-cttgaacctcAaacaattga-3' | 48 | 100 |
| | 5'-cttgaacctcAaacaattg-3' | 46.4 | 101 |
| | 5'-cttgaacctcAaacaatt-3' | 44.3 | 102 |
| Probe (3) for NAT2*7 | 5'-cccaaacctggtgatgAatcc-3' | 54.9 | 103 |
| | 5'-ccaaacctggtgatgAatcc-3' | 52.5 | 104 |
| | 5'-caaacctggtgatgAatcc-3' | 49.9 | 105 |
| | 5'-aaacctggtgatgAatcc-3' | 48 | 106 |
| | 5'-aacctggtgatgAatcc-3' | 47 | 107 |
| | 5'-acctggtgatgAatcc-3' | 45.8 | 108 |

Each probe (1) indicated in the above table consists of a sequence complementary to that of a region having C at base 1063 in SEQ ID NO: 1, and the capitalized base indicates base complementary to base 1063 in SEQ ID NO: 1. In each probe (1), the capitalized base can be replaced by "r", and the "r" may be either G or A. Each probe (2) indicated in the above table consists of a sequence identical to that of a region having A at base 1312 in SEQ ID NO: 1, and the capitalized base indicates base 1312 in SEQ ID NO: 1. In each probe (2), the capitalized base can be replaced by "r", and the "r" may be either G or A. Each probe (3) indicated in the above table consists of a sequence identical to that of a region having A at base 1579 in SEQ ID NO: 1, and the capitalized base indicates base 1579 in SEQ ID NO: 1. In each probe (3), the capitalized base can be replaced by "r", and the "r" may be either G or A. As described above, specific examples of the probe according to the present invention may be strands complementary to oligonucleotides indicated in the above table.

The aforementioned probes are examples and the present invention is not limited thereto. With respect to the probe for NAT2*5, a preferable probe among the probes (1) is at least one oligonucleotide selected from the group consisting of oligonucleotide consisting of the base sequence of SEQ ID NO: 90, SEQ ID NO: 91, SEQ ID NO: 118, and SEQ ID NO: 122. Further, with respect to the probe for NAT2*5, a so-called wild-type detecting probe preferably is used in combination with a so-called mutation type detecting probe. In this state, the wild-type detecting probe is, for example, a probe for detecting a sequence to be detected including T at base 1063 in SEQ ID NO:1 (a sense strand) or a strand complementary thereto (an antisense strand), and the mutation type detecting probe is a probe for detecting a sequence to be detected including C at base 1063 in SEQ ID NO:1 (a sense strand) or a strand complementary thereto (an antisense strand). In the present invention, at least one oligonucleotide consisting of the base sequence of SEQ ID NOs: 87 to 92 and 116 (the mutation type detecting probe) preferably is used in combination with at least one oligonucleotide consisting of the base sequence of SEQ ID NOs: 117 to 123 (the wild-type detecting probe), and more preferably, oligonucleotide consisting of the base sequence of SEQ ID NO: 90 or SEQ ID NO: 91 (the mutation type detecting probe) is used in combination with oligonucleotide consisting of the base sequence of SEQ ID NO: 118 or SEQ ID NO: 122 (the wild-type detecting probe). In this manner, when the wild-type detecting probe is used in combination with the mutation type detecting probe, for example, the Tm value of perfect match of each probe is preferably set at different values.

With respect to the probe for NAT2*6, oligonucleotide consisting of the base sequence of SEQ ID NO: 99 is preferable. With respect to the probe for NAT2*7, oligonucleotide consisting of the base sequence of SEQ ID NO: 105 or SEQ ID NO: 107 is preferable.

When two or more of these probes are used, as described above, it is preferable that they be labeled with different fluorescent dyes (fluorescent dyes that are detected at different wavelengths). For instance, when the probes indicated in the above table are guanine quenching probes, it is preferable that in each probe for NAT2*5 (probe (1)) and each probe for NAT2*7 (probe (3)), cytosine at the 3' end thereof be labeled with a fluorescent dye (for instance, BODIPY FL or TAMRA) as described above and in each probe for NAT2*6 (probe (2)), cytosine at the 5' end thereof be labeled with a fluorescent dye (for instance, Pacific Blue) as described above. Furthermore, a probe with the 5' end labeled with a fluorescent dye may have the 3' end, to which a phosphate group further may be added, in order to prevent the probe itself from elongating. Moreover, when the wild-type detecting probe and the mutation type detecting probe are used as described above, each fluorescent dye may be the same or different.

Next, with respect to the detection method of the present invention, a method of detecting three polymorphisms, NAT2*5, NAT2*6, and NAT2*7, in the NAT2 gene using the following probes is described as an example. However, the present invention is not limited thereto.

### <Probes>

| Probe for NAT2*5 | |
|---|---|
| 5'-tgccgtcaGtggtcac-(BODIPY FL)-3' | (SEQ ID NO: 90), |
| 5'-gccgtcaGtggtcac-(BODIPY FL)-3' | (SEQ ID NO: 91), |
| 5'-cctgccgtcaAtggtcac-(BODIPY FL)-3' | (SEQ ID NO: 118), or |
| 5'-ccgtcaAtggtcac-(BODIPY FL)-3' | (SEQ ID NO: 122) |

| Probe for NAT2*6 | |
|---|---|
| 5'-(Pacific Blue)-cttgaacctcAaacaattgaa-P-3' | (SEQ ID NO: 99) |

| Probe for NAT2*7 | |
|---|---|
| 5'-caaacctggtgatgAatcc-(TAMRA)-3' | (SEQ ID NO: 105), or |
| 5'-aacctggtgatgAatcc-(TAMRA)-3' | (SEQ ID NO: 107) |

First, using a reaction solution containing the aforementioned three labeled probes added thereto, PCR was performed as described above, and thereby the three regions of the NAT2 gene are amplified at the same time in the same reaction solution. The reaction solution contains, for example, a primer set for amplifying the NAT2 gene of the present invention, DNA polymerase, dNTP, a sample containing nucleic acid to serve as a template, and the aforementioned three probes. In addition to them, various additives that can be used for amplifying nucleic acid may be contained.

Next, the amplification products thus obtained are dissociated and then single-stranded DNA obtained through dissociation is hybridized with the labeled probes. This can be carried out through, for example, a change in the temperature of the reaction solution.

The heating temperature in the dissociation step is not particularly limited as long as it allows the amplification products to be dissociated. It is, for example, 85 to 95°C. The heating time also is not particularly limited and generally is 1 second to 10 minutes and preferably 1 second to 5 minutes.

The dissociated single-stranded DNAs can be hybridized with the labeled probes by, for example, decreasing the heating temperature employed in the dissociation step after the dissociation step. The temperature condition is, for example, 40 to 50°C.

The temperature of the reaction solution is changed and thereby signal values that indicate the melting states of hybridization products between the amplification products and the labeled probes are measured. Specifically, for example, the reaction solution (the hybridization products between the single-stranded DNAs and the labeled probes) is heated, and thereby the change in the signal values accompanying the temperature rise is measured. As described above, when using, for example, a probe (guanine quenching probe), in which the base C at the end has been labeled, fluorescence decreases (or quenches) in the state where the probe has been hybridized with the single-stranded DNA, while fluorescence is emitted in the state where the probe has been dissociated. Accordingly, for example, the hybridization product in which the fluorescence has decreased (or quenched) is heated gradually and the increase in fluorescence intensity accompanying the temperature rise may be measured.

The temperature range in which the change in fluorescence intensity is to be measured is not particularly limited. For example, the start temperature is room temperature to 85°C and preferably 25 to 70°C, while the end temperature is, for example, 40 to 105°C. Furthermore, the rate of temperature rise is not particularly limited and is, for example, 0.1 to 20°C/sec and preferably 0.3 to 5°C/sec.

Next, the Tm value is determined by analyzing a change in the signal. Specifically, the amount of change in the fluorescence intensity per unit time at each temperature (-d fluorescence intensity increase/dt) is calculated from the fluorescence intensity obtained and the temperature at which the lowest value is obtained is determined as the Tm value. It is also possible to determine, as the Tm value, the point at which the amount of increase in the fluorescence intensity per unit time (fluorescence intensity increase/t) is the highest. On the contrary, the amount of decrease in the fluorescence intensity is measured when the labeled probe used is not a quenching probe but a probe that does not exhibit a signal independently but exhibits a signal after hybridization.

In the present invention, in order to detect three polymorphisms, NAT2*5, NAT2*6, and NAT2*7, the respective Tm values are determined under conditions according to the respective labels of the three probes. BODIPY FL, a probe for NAT2*5, can be detected with, for example, a detection wavelength of 515 to 555 nm, TAMRA, Pacific Blue, a probe for NAT2*6, with, for example, a detection wavelength of 450 to 480 nm, and TAMRA, a probe for NAT2*7, with, for example, a detection wavelength of 585 to 700 nm.

From such Tm values, the genotypes in the respective sites to be detected are determined. In the Tm analysis, the case of a perfectly complementary hybrid (match) results in a higher Tm value indicating dissociation than that obtained in the case of a hybrid including a different single base (mismatch). Accordingly, when with respect to the probe, the Tm value obtained in the case of a perfectly complementary hybrid and the Tm value obtained in the case of a hybrid including a different single base are determined beforehand, the genotype at each site to be detected can be determined. For example, in the case where the base located at the site to be detected is assumed to be of a mutation type (with, for instance, C at base 1063 in SEQ ID NO: 1), when using a probe complementary to the sequence to be detected containing the base, the polymorphism of the amplification product can be judged as a mutation type if the Tm value of the resultant hybrid is equal to the Tm value of a perfectly complementary hybrid. Furthermore, the polymorphism of the amplification product can be judged as a wild-type (with, for example, T at base 1063 in SEQ ID NO: 1) if the Tm value of the resultant hybrid is equal to the Tm value of the hybrid including a different single base (i.e. a lower value than the Tm value of the perfectly complementary hybrid). Moreover, when both the Tm values are detected, it can be judged as heterozygote. Thus, the genotypes of the polymorphisms, NAT2*5, NAT2*6, and NAT2*7, can be judged from the three Tm values with respect to the respective labeled probes.

In the present invention, for example, a change in the signal during hybridization may be measured instead of the method in which the temperature of a reaction solution containing the probes is increased (a hybridization product is heated) and a change in the signal accompanying the temperature rise is measured as described above. In other words, when the temperature of the reaction solution containing the aforementioned probes is decreased to form hybridization products, the change in the signal accompanying the temperature decrease may be measured.

Specifically, when using a labeled probe that exhibits a signal independently but does not exhibit a signal after hybridization (for example, a guanine quenching probe), the labeled probe emits fluorescence in the state where single-stranded DNA and the probe are dissociated, but the fluorescence decreases (or quenches) when a hybrid is formed through temperature decrease. Accordingly, for example, the temperature of the reaction solution is decreased gradually and the decrease in fluorescence intensity accompanying the temperature decrease may be measured. On the other hand, when using a labeled probe that does not exhibit a signal independently but exhibits a signal after hybridization, the labeled probe does not emit fluorescence in the state where single-stranded DNA and the probe are dissociated, but the fluorescence is emitted when a hybrid is formed through temperature decrease. Accordingly, for example, the temperature of the reaction solution is decreased gradually and thereby the increase in fluorescence intensity accompanying the temperature decrease may be measured.

When one or two of the three types of polymorphisms (NAT2*5, NAT2*6, and NAT2*7) in the NAT2 gene are to be analyzed, for instance, a primer set for amplifying the NAT2 gene of the present invention may be used that includes one or two types of primer sets corresponding to the target regions that are selected from the primer sets (1) to (3), and furthermore, one or two probes that hybridize to target sites to be detected may be used.

Next, examples of the present invention are described. However, the present invention is not limited by the following examples.

### [Example 1]

Blood was collected from four subjects using heparin lithium blood collection tubes (Samples 1 to 4). Subsequently, 10 µL of blood thus obtained and 90 µL of distilled water were mixed together. Further, 10 µL of this mixture and 90 µL of distilled water were mixed together. Thereafter, 10 µL of the mixture was added to 40 µL of PCR reaction solution having the following composition, and then PCR was performed using a thermal cycler. Conditions for PCR were as follows. That is, after treating at 95°C for 60 seconds, one cycle of treatment at 95°C for 1 second and at 60°C for 10 seconds was repeated for 50 cycles, and further it was treated at 95°C for 1 second and at 40°C for 60 seconds. Subsequently, the PCR reaction solution was heated from 40°C to 95°C at a rate of temperature rise of 1°C/3 seconds, and the change in fluorescence intensity over time was measured. The measurement wavelength was 450 to 480 nm (for detection of the fluorescent dye, Pacific Blue), 515 to 555 nm (for detection of the fluorescent dye, BODIPY FL), and 585 to 700 nm (for detection of the fluorescent dye, TAMRA). The time required for 50 cycles of PCR was approximately one hour.

**[Table 6]**

| <PCR reaction solution; unit: µl> | |
|---|---|
| Distilled water | 17.375 |
| 5% NaN₃ | 0.5 |
| 20% BSA | 1 |
| 40% Glycerol | 3.125 |
| 10 × Gene Taq buffer * | 5 |
| 2.5 mM dNTPs | 4 |
| 100 mM MgCl₂ | 0.5 |
| 5 µM probe 1 for NAT2*5 | 1 |
| 5 µM probe 2 for NAT2*5 | 3 |
| 5 µM probe for NAT2*6 | 1.5 |
| 5 µM probe for NAT2*7 | 0.5 |
| 100 µM NAT2*5 F1 primer | 0.5 |
| 100 µM NAT2*5 R1 primer | 0.25 |
| 100 µM NAT2*6 F2 primer | 0.25 |
| 100 µM NAT2*6 R2 primer | 0.5 |
| 100 µM NAT2*7 F3 primer | 0.25 |
| 100 µM NAT2*7 R3 primer | 0.5 |
| 5 U/µl Gene Taq FP * | 0.25 |
| Total | 40 µL |

| | |
|---|---|
| * Trade name, Gene Taq Fp: manufactured by Nippon Gene Co., Ltd. | |

| <Probes> | | |
|---|---|---|
| Probe 1 for NAT2*5 | | |
| | 5'-tgccgtcaGtggtcac-(BODIPY FL)-3' | (SEQ ID NO: 90) |
| Probe 2 for NAT2*5 | | |
| | 5'-tgccgtcaGtggtcac-P-3' | (SEQ ID NO: 90) |
| Probe for NAT2*6 | | |
| | 5'-(Pacific Blue)-cttgaacctcAaacaattgaa-P-3' | (SEQ ID NO: 99) |
| Probe for NAT2*7 | | |
| | 5'-caaacctggtgatgAatcc-(TAMRA)-3' | (SEQ ID NO: 105) |

| <Primer set> | | |
|---|---|---|
| NAT2*5 F1 primer | | |
| | 5'-cagttaacaaatacagcactggcatgg-3' | (SEQ ID NO: 7) |
| NAT2*5 R1 primer | | |
| | 5'-acatctgggaggagcttccag-3' | (SEQ ID NO: 18) |
| NAT2*6 F2 primer | | |
| | 5'-ctcatctcctgccaaagaagaaac-3' | (SEQ ID NO: 33) |
| NAT2*6 R2 primer | | |
| | 5'-gatgtggttataaatgaagatgttggagac-3' | (SEQ ID NO: 48) |
| NAT2*7 F3 primer | | |
| | 5'-aaatatatttaagatttccttggggagaaat-3' | (SEQ ID NO: 60) |
| NAT2*7 R3 primer | | |
| | 5'-tgagttgggtgatacatacacaaggg-3' | (SEQ ID NO: 81) |

The Tm value of a hybrid that matches with the probe for NAT2*5 is 66.0°C and that of a hybrid that mismatches therewith is 58.0°C, the Tm value of a hybrid that matches with the probe for NAT2*6 is 61.0°C and that of a hybrid that mismatches therewith is 53.0°C, and the Tm value of a hybrid that matches with the probe for NAT2*7 is 63.0°C and that of a hybrid that mismatches therewith is 56.0°C.

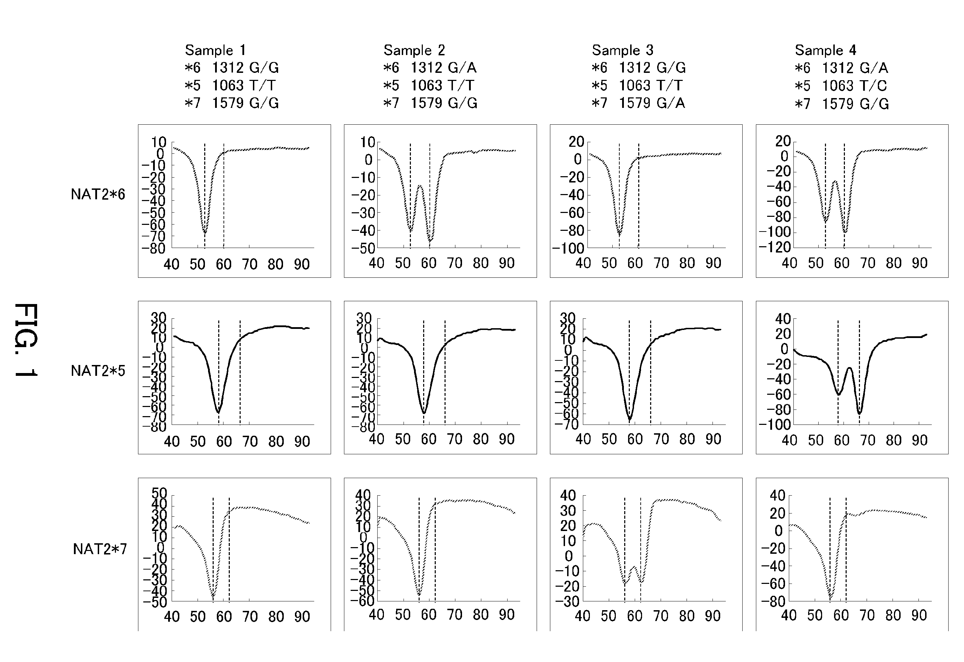

Results of samples 1 to 4 are indicated in FIG. 1. This figure shows graphs of Tm analysis that indicate the changes in fluorescence intensity accompanying temperature rise. The differential value of the vertical axis indicates "-d fluorescence intensity increase/dt", while the horizontal axis indicates temperature (the same applies below). As shown in this graph, the genotypes of NAT2*5, NAT2*6, and NAT2*7 in each sample were determined from the peaks of the signals. In order to support the results of these examples, with respect to four subjects, the genotypes of NAT2*5, NAT2*6, and NAT2*7 were confirmed by the RFLP method and the sequencing method. As a result, the same results as those obtained in the example were obtained. Accordingly, the use of a primer set of the present invention made it possible to amplify three regions of the NAT2 gene simultaneously in the same reaction solution using a whole blood sample that had not been pretreated and to analyze the three types of polymorphisms using the same reaction solution.

### [Example 2]

Blood was collected from three subjects using EDTA blood collection tubes (Samples 1 to 3). Subsequently, 10 µL of blood thus obtained and 70 µL of diluent A described below were mixed together. Further, 10 µL of this mixture and 70 µL of diluent B described below were mixed together. Subsequently, 10 µL of the mixture thus obtained was heat-treated at 95°C for five minutes. Thereafter, this was added to 46 µL of PCR reaction solution having the following composition, and then PCR was performed using a thermal cycler. Conditions for PCR were as follows. That is, after treating at 95°C for 60 seconds, one cycle of treatment at 95°C for 1 second and at 65°C for 15 seconds was repeated for 50 cycles, and further it was treated at 95°C for 1 second and at 40°C for 60 seconds. Subsequently, the PCR reaction solution was heated from 40°C to 75°C at a rate of temperature rise of 1°C/3 seconds, and the change in fluorescence intensity over time was measured. The measurement wavelength was 450 to 480 nm (for detection of the fluorescent dye, Pacific Blue), 515 to 555 nm (for detection of the fluorescent dye, BODIPY FL), and 585 to 700 nm (for detection of the fluorescent dye, TAMRA).

### <Diluent A>

10 mM Tris-HCl (pH 8), 0.1 mM EDTA, 0.05% NaN₃, 0.3% SDS

### <Diluent B>

10 mM Tris-HCl (pH 8), 0.1 mM EDTA, 0.05% NaN₃

**[Table 7]**

| <PCR reaction solution; unit: µl> | |
|---|---|
| Distilled water | 14.5 |
| 5% NaN₃ | 0.5 |
| 20% BSA | 0.5 |
| 40% Glycerol | 12.5 |
| 10 × Gene Taq buffer * | 5 |
| 2.5 mM dNTPs | 4 |
| 100 mM MgCl₂ | 0.5 |
| 5 µM probe 1 for NAT2*5 | 1 |
| 5 µM probe 2 for NAT2*5 | 1 |
| 5 µM probe for NAT2*6 | 2 |
| 5 µM probe for NAT2*7 | 2 |
| 100 µM NAT2*5 F1 primer | 0.5 |
| 100 µM NAT2*5 R1 primer | 0.25 |
| 100 µM NAT2*6 F2 primer | 0.25 |
| 100 µM NAT2*6 R2 primer | 0.5 |
| 100 µM NAT2*7 F3 primer | 0.25 |
| 100 µM NAT2*7 R3 primer | 0.5 |
| 5 U/µl Gene Taq FP * | 0.25 |
| Total | 46 µL |

| | |
|---|---|
| * Trade name, Gene Taq Fp: manufactured by Nippon Gene Co., Ltd. | |

| <Probes> | | |
|---|---|---|
| Probe1 for NAT2*5 | | |
| | 5'-gccgtcaGtggtcac-(BODIPY FL)-3' | (SEQ ID NO: 91) |
| Probe 2 for NAT2*5 | | |
| | 5'-ccgtcaAtggtcac-(BODIPY FL)-3' | (SEQ ID NO: 118) |
| Probe for NAT2*6 | | |
| | 5'-(Pacific Blue)-cttgaacctcAaacaattgaa-P-3' | (SEQ ID NO: 99) |
| Probe for NAT2*7 | | |
| | 5'-aacctggtgatgAatcc-(TAMRA)-3' | (SEQ ID NO: 107) |

| <Primer set> | | |
|---|---|---|
| NAT2*5 F1 primer | | |
| | 5'-tccagttaacaaatacagcactggcatgg-3' | (SEQ ID NO: 7) |
| NAT2*5 R1 rimer | | |
| | 5'-ccacatctgggaggagcttccag-3' | (SEQ ID NO: 18) |
| NAT2*6 F2 primer | | |
| | 5'-agaatttcttaattctcatctcctgccaaagaagaaac-3' | (SEQ ID NO: 33) |
| NAT2*6 R2 primer | | |
| | 5'-gaacaaaatgatgtggttataaatgaagatgttggagac-3' | (SEQ ID NO: 48) |
| NAT2*7 F3 primer | | |
| | 5'-agtgctgaaaaatatatttaagatttccttggggagaaat-3' | (SEQ ID NO: 60) |
| NAT2*7 R3 primer | | |
| | 5'-tgataattagtgagttgggtgatacatacacaaggg-3' | (SEQ ID NO: 81) |

The Tm value of a hybrid that matches with the probe for NAT2*5 is 63°C and that of a hybrid that mismatches therewith is 56°C, the Tm value of a hybrid that matches with the probe for NAT2*6 is 58°C and that of a hybrid that mismatches therewith is 50.5°C, and the Tm value of a hybrid that matches with the probe for NAT2*7 is 58°C and that of a hybrid that mismatches therewith is 49°C.

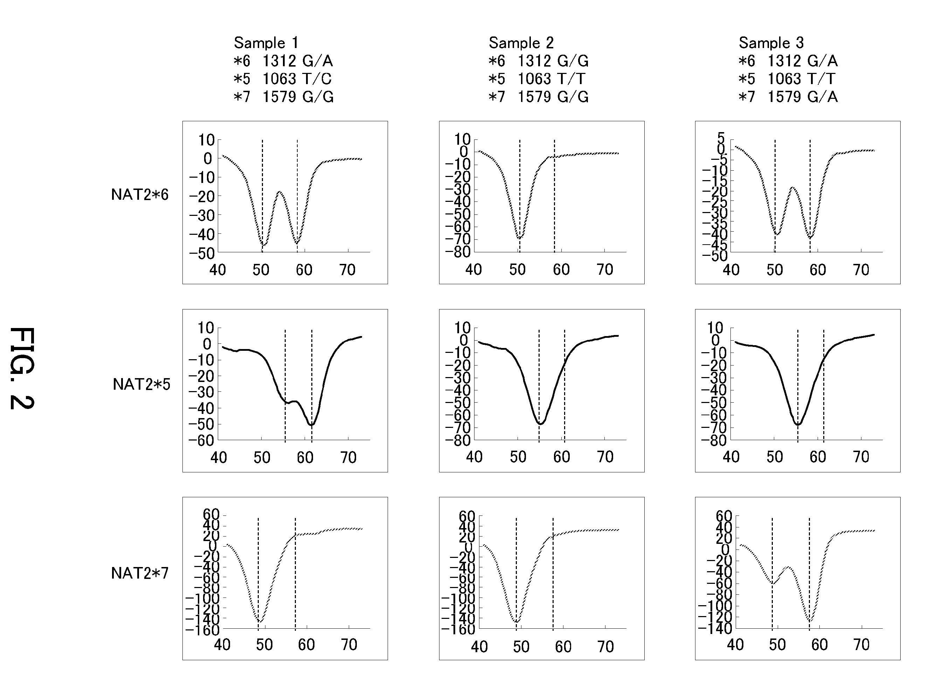

Results of Samples 1 to 3 are indicated in FIG. 2. FIG. 2 shows graphs of Tm analysis that indicate the changes in fluorescence intensity accompanying temperature rise. The differential value of the vertical axis indicates "-d fluorescence intensity increase/dt", while the horizontal axis indicates temperature. As shown in these graphs, the genotypes of NAT2*5, NAT2*6, and NAT2*7 in each sample were determined from the peaks of the signals. In order to support the results of these examples, with respect to three subjects, the polymorphisms of NAT2*5, NAT2*6, and NAT2*7 were confirmed by the RFLP method and the sequencing method. As a result, the same results as those obtained in the example were obtained. Accordingly, the use of a primer set of the present invention made it possible to amplify three regions of the NAT2 gene simultaneously in the same reaction solution using a whole blood sample that had not been pretreated and to analyze the three types of polymorphisms using the same reaction solution.

### Industrial Applicability

As described above, the primer set of the present invention makes it possible to specifically and efficiently amplify a region including a site where a particular polymorphism (NAT2*5, NAT2*6, and NAT2*7) is generated in the NAT2 gene. This allows time and cost to be reduced, which is different from the conventional methods as described above. Furthermore, since the region including a site to be detected of a polymorphism is amplified specifically, for example, the use of a probe complementary to a sequence to be detected including the site to be detected makes it possible to perform Tm analysis directly using the aforementioned reaction solution to type the polymorphism. Moreover, since amplification and typing can be carried out using one reaction solution, the operation can be automated. The use of the primer set of the present invention allows a pretreatment to be omitted even in the case of, for example, a contaminated sample (for instance, whole blood or oral mucosa), and therefore the amplification reaction can be carried out quicker and more easily. Furthermore, when the primer set of the present invention is used, the amplification reaction can be carried out with higher amplification efficiency as compared to conventional cases and thus the reaction time can also be shortened. According to the primer set of the present invention, the reagent including the same, as well as the method of manufacturing an amplification product using them, since the polymorphism in the NAT2 gene can be analyzed quickly and simply, it can be said that they are considerably effective in the field of medicine.
## Claims

1. A primer set for amplifying the NAT2 gene by a gene amplification method,
wherein the primer set includes at least one selected from the group consisting of the following primer sets (1) to (3):
Primer set (1):
a primer set of a pair of primers including a forward primer composed of the following oligonucleotide (F1) and a reverse primer composed of the following oligonucleotide (R1):
(F1): at least one oligonucleotide having a sequence identical to that of a region extending from guanine (G) at base 1038 to be considered as the first base to any one of the 20^{th} to 32^{nd} bases in the direction toward the 5' end in the base sequence of SEQ ID NO: 1, with the guanine (G) being the 3' end, and
(R1): at least one oligonucleotide complementary to a region extending from cytosine (C) at base 1096 to be considered as the first base to any one of the 17^{th} to 24^{th} bases in the direction toward the 3' end in the base sequence of SEQ ID NO: 1, with guanine (G) complementary to the cytosine (C) at base 1096 being the 3' end,
Primer set (2):
a primer set of a pair of primers including a forward primer composed of the following oligonucleotide (F2) and a reverse primer composed of the following oligonucleotide (R2):
(F2): at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
(R2): at least one oligonucleotide selected from:
at least one oligonucleotide complementary to a region extending from guanine (G) at base 1355 to be considered as the first base to any one of the 25^{th} to 40^{th} bases in the direction toward the 3' end in the base sequence of SEQ ID NO: 1, with cytosine (C) complementary to the guanine (G) at base 1355 being the 3' end, and
at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end, and
Primer set (3):
a primer set of a pair of primers including a forward primer composed of the following oligonucleotide (F3) and a reverse primer composed of the following oligonucleotide (R3):
(F3): at least one oligonucleotide selected from:
at least one oligonucleotide having a sequence identical to that of a region extending from thymine (T) at base 1556 to be considered as the first base to any one of the 21^{st} to 40^{th} bases in the direction toward the 5' end in the base sequence of SEQ ID NO: 1, with the thymine (T) being the 3' end, and
at least one oligonucleotide having a sequence identical to that of a region extending from cytosine (C) at base 1278 to be considered as the first base to any one of the 20^{th} to 38^{th} bases in the direction toward the 5' end in the base sequence of SEQ ID NO: 1, with the cytosine (C) being the 3' end, and
(R3): at least one oligonucleotide complementary to a region extending from cytosine (C) at base 1614 to be considered as the first base to any one of the 21^{st} to 36^{th} bases in the direction toward the 3' end in the base sequence of SEQ ID NO: 1, with guanine (G) complementary to the cytosine (C) at base 1614 being the 3' end.

2. The primer set for amplifying the NAT2 gene according to claim 1, wherein the primer sets (1) to (3) are the following primer sets (1') to (3'), respectively:
Primer set (1'):
a primer set of a pair of primers including a forward primer composed of the following oligonucleotide (F1') and a reverse primer composed of the following oligonucleotide (R1'):
(F1'): at least one oligonucleotide selected from oligonucleotide consisting of the base sequence of SEQ ID NO: 5 and oligonucleotide consisting of the base sequence of SEQ ID NO: 7, and
(R1') at least one oligonucleotide selected from oligonucleotide consisting of the base sequence of SEQ ID NO: 16 and oligonucleotide consisting of the base sequence of SEQ ID NO: 18,
Primer set (2'):
a primer set of a pair of primers including a forward primer composed of the following oligonucleotide (F2') and a reverse primer composed of the following oligonucleotide (R2'):
(F2'): at least one oligonucleotide selected from oligonucleotide consisting of the base sequence of SEQ ID NO: 33 and oligonucleotide consisting of the base sequence of SEQ ID NO: 109, and
(R2') at least one oligonucleotide selected from oligonucleotide consisting of the base sequence of SEQ ID NO: 39 and oligonucleotide consisting of the base sequence of SEQ ID NO: 48, and
Primer set (3'):
a primer set of a pair of primers including a forward primer composed of the following oligonucleotide (F3') and a reverse primer composed of the following oligonucleotide (R3'):
(F3'): at least one oligonucleotide selected from oligonucleotide consisting of the base sequence of SEQ ID NO: 60 and oligonucleotide consisting of the base sequence of SEQ ID NO: 113, and
(R3') at least one oligonucleotide selected from oligonucleotide consisting of the base sequence of SEQ ID NO: 71 and oligonucleotide consisting of the base sequence of SEQ ID NO: 81.

3. The primer set for amplifying the NAT2 gene according to claim 1, wherein the primer set for amplifying the NAT2 gene is a primer set for amplifying the NAT2 gene in a biological sample.

4. The primer set for amplifying the NAT2 gene according to claim 3, wherein the biological sample is whole blood.

5. A reagent for amplifying the NAT2 gene by a gene amplification method,
wherein the reagent comprises a primer set for amplifying the NAT2 gene according to claim 1.

6. The reagent for amplifying the NAT2 gene according to claim 5, further comprising a probe that can hybridize to a site to be detected in the NAT2 gene.

7. The reagent for amplifying the NAT2 gene according to claim 6, wherein the probe is at least one probe selected from the group consisting of the following oligonucleotides (P1') to (P3'):
(P1') at least one oligonucleotide selected from oligonucleotide consisting of the base sequence of SEQ ID NO: 90, oligonucleotide consisting of the base sequence of SEQ ID NO: 91, oligonucleotide consisting of the base sequence of SEQ ID NO: 118, and oligonucleotide consisting of the base sequence of SEQ ID NO: 122,
(P2') oligonucleotide consisting of the base sequence of SEQ ID NO: 99, and
(P3') at least one oligonucleotide selected from oligonucleotide consisting of the base sequence of SEQ ID NO: 105, and oligonucleotide consisting of the base sequence of SEQ ID NO: 107.

8. The reagent for amplifying the NAT2 gene according to claim 6, wherein the probe is a fluorescently-labeled probe.

9. A method of manufacturing an amplification product of the NAT2 gene by a gene amplification method,
wherein the method comprises the following process (I):
(I) amplifying the NAT2 gene in a reaction solution using a primer set for amplifying the NAT2 gene according to claim 1, with nucleic acid contained in a sample being used as a template.

10. The method of manufacturing an amplification product according to claim 9, wherein a probe that can hybridize to a site to be detected in the NAT2 gene further is added to the reaction solution in the process (I).

11. The method of manufacturing an amplification product according to claim 10, wherein the probe is at least one probe selected from the group consisting of the following oligonucleotides (P1') to (P3'):
(P1') at least one oligonucleotide selected from oligonucleotide consisting of the base sequence of SEQ ID NO: 90, oligonucleotide consisting of the base sequence of SEQ ID NO: 91, oligonucleotide consisting of the base sequence of SEQ ID NO: 118, and oligonucleotide consisting of the base sequence of SEQ ID NO: 122,
(P2') oligonucleotide consisting of the base sequence of SEQ ID NO: 99, and
(P3') at least one oligonucleotide selected from oligonucleotide consisting of the base sequence of SEQ ID NO: 105, and oligonucleotide consisting of the base sequence of SEQ ID NO: 107.

12. The method of manufacturing an amplification product according to claim 10, wherein the probe is a fluorescently-labeled probe.

13. The method of manufacturing an amplification product according to claim 12, wherein the method further comprises the following process (II):
(II) measuring fluorescence intensity of a fluorescent label contained in the fluorescently-labeled probe in the reaction solution.

14. The method of manufacturing an amplification product according to claim 9, wherein the sample is a biological sample.

15. The method of manufacturing an amplification product according to claim 14, wherein the biological sample is whole blood.

16. The method of manufacturing an amplification product according to claim 15, wherein the ratio of the whole blood sample to be added to the reaction solution is 0.1 to 0.5 vol%.

17. A polymorphism analysis method of analyzing a polymorphism of a site to be detected in the NAT2 gene, wherein the method comprises the following processes (i) to (iv):
(i) amplifying a region including a site to be detected in the NAT2 gene in a reaction solution by a method of manufacturing an amplification product according to claim 9,
(ii) preparing a reaction solution that contains the amplification product obtained in the process (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

18. The polymorphism analysis method according to claim 17, wherein in the process (i), a probe that can hybridize to the site to be detected is added to the reaction solution prior to an amplification reaction.


[Sequence Table] TF07038-01.ST25.txt

SEQUENCE LISTING

<110>  ARKRAY, Inc.

<120>  Primer set and agent for amplification of NAT2 gene and use thereof

<130>  P24715

<150>  JP2006-322956
<151>  2006-11-30

<150>  JP2007-232612
<151>  2007-09-07

<160>  123

<170>  PatentIn version 3.1

<210>  1
<211>  6464
<212>  DNA
<213>  Homo sapiens

<400>  1
gaattcagtg tctctccctg tgcacccact aacccctctt tttgttttca ccaggcactt      60

accacaatct aacagactgc atgttttatc catttattca gtttcctatt tgtgtccctt     120

caactcccat taaaatataa tatttttgag ggcaagcaag tactagaaca ataggaaaca     180

catcaagagt attctgtaaa ctatttcttg aatcaatcag tgaatgaatg aattaatcaa     240

tatatttttt gagtgaggag ctttgtgtta ggtacagcta aatgggaaat caagtgggtc     300

atgtaccatg aataccatat actctactgt ataattctcc tgcttatatc agaaactgtt     360

tataagccta ttataattga taccaattgg aatctctttt ttactcatca ccaagaacac     420

cacaaacaag ttgtttacca tttggctcct tatttaatct ggatttccaa ctcctcatgc     480

ttaaaagacg gaagatacaa taatactttc cttacagggt tctgagacta ctaagagaac     540

ttatgcatgt aaaagggatt catgcagtag aaatactaac aaaagaatta ctatgacaga     600

tacttataac cattgtgttt ttacgtattt aaaatacgtt atacctataa ttagtcacac     660

gaggaaatca aatgctaaag tatgatatgt ttttatgttt tgtttttctt gcttagggga     720

tcatggacat tgaagcatat tttgaaagaa ttggctataa gaactctagg aacaaattgg     780

acttggaaac attaactgac attcttgagc accagatccg ggctgttccc tttgagaacc     840

ttaacatgca ttgtgggcaa gccatggagt tgggcttaga ggctattttt gatcacattg     900

taagaagaaa ccggggtggg tggtgtctcc aggtcaatca acttctgtac tgggctctga     960

ccacaatcgg ttttcagacc acaatgttag gagggtattt ttacatccct ccagttaaca    1020

aatacagcac tggcatggtt caccttctcc tgcaggtgac cattgacggc aggaattaca    1080

ttgtcgatgc tgggtctgga agctcctccc agatgtggca gcctctagaa ttaatttctg    1140

ggaaggatca gcctcaggtg ccttgcattt tctgcttgac agaagagaga ggaatctggt    1200

acctggacca aatcaggaga gagcagtata ttacaaacaa agaatttctt aattctcatc    1260

tcctgccaaa gaagaaacac caaaaaatat acttatttac gcttgaacct cgaacaattg    1320

aagattttga gtctatgaat acatacctgc agacgtctcc aacatcttca tttataacca    1380

catcattttg ttccttgcag accccagaag gggtttactg tttggtgggc ttcatcctca 1440
cctatagaaa attcaattat aaagacaata cagatctggt cgagtttaaa actctcactg 1500
aggaagaggt tgaagaagtg ctgaaaaata tatttaagat ttccttgggg agaaatctcg 1560
tgcccaaacc tggtgatgga tcccttacta tttagaataa ggaacaaaat aaacccttgt 1620
gtatgtatca cccaactcac taattatcaa cttatgtgct atcagatatc ctctctaccc 1680
tcacgttatt ttgaagaaaa tcctaaacat caaatacttt catccataaa aatgtcagca 1740
tttattaaaa aacaataact ttttaaagaa acataaggac acattttcaa attaataaaa 1800
ataaaggcat tttaaggatg gcctgtgatt atcttgggaa gcagagtgat tcatgctaga 1860
aaacatttaa tattgattta ttgttgaatt catagtaaat ttttactggt aaatgaataa 1920
agaatattgt ggaaaactca ctgtctctaa agtttatgaa aacattgttg gctaatatat 1980
tgtgaatcaa agtttttctt tagacgactt aggatattat ggggctaggc attttttcct 2040
caatagagtc ttcctctcat cctctttctt gtctcctagt tacattcttt tacttccatc 2100
catactttgc cacaagagaa ggaacatgag ctttattgtg tagatctgat ttgaaatcct 2160
gtggacacgg ggtgaattac ttttaaaatc tgtggctctg attcctcaaa gataaaatgc 2220
aaataatatt tatataattc actgccagat ataaattttc aaaactattt gttatatgga 2280
tgaataacat cattaatatt gtggttgctg ggccagcatt tgccaaaagt tctccttcca 2340
ttttgcttta ttttcctgta acttgaaatt ctggtcctac tgtcatctgc ctgcttcttc 2400
cttaattaaa tattgatagg atatcagatg tctcggatct gagagtgtgc cttgtgattc 2460
aaaatctgaa tctttactta tccataactc agattttctg tttgtaaatt ccagtatcag 2520
ggctatagtt taaactgcag atttgttctt aacactattc tccctcttcg actcgtgatg 2580
actataataa tcttaagaga aaagcagaca ttagaatgaa taaatattca ttaggagaat 2640
aaattacatt gaagcatcag tattttaggc agcagtgtaa tagttgggag atactggtga 2700
gtgtagatat cctaggaaga ggtggatagg agatctggcc tcagtgggaa ggacaaatga 2760
aagacatata gcaatatttg agagcttgtc atctttctta cctattagcc ttgttcagct 2820
ctcctgctat cttgttgcaa tgccaggtca ccactggtgc tcctaggcag acccaagttt 2880
ctcacattct gagcaagatc acatcacagg aggatgtggt ggcaaaaaac aaaaatgaaa 2940
acaaaacaat caaacaaaaa accagataaa aatgtggctc aagtatgaga tacagttgta 3000
tcaatgaatc aagtaaatta tattgcacac gagtatctga acctaattca gttgtttgtt 3060
cttgattata tatatgtaca tatggaaaaa gcaaaaatat gttcagagag attcagaaat 3120
acacaatttc gcttccaggt tgaagccttc tgcccttatt atgcaatgtt accttttctc 3180
taatgaaatc taaatgagtg aagaagaatc tcaccaattg atttggccag agattttaag 3240
gtgcctctta attgtttgtg tttgtccaaa cccatgtctc tgtttttggt ggccccctgg 3300
agactaggat gtggcatatc ttggtagaac tctgagataa gtaaggtaga aaccaaacct 3360
tctagacgta actgggaagg taggatcttg gatgtatatt ccagttcttc tattttcaag 3420

gtgaagctga gtgtgtgttt ttatctgcca ctctctctgc tgaaagccag ggagattata 3480
tggggcaagt acccatactg gtgttcaggc ggcagcctct gatcctagga gatacctatt 3540
gaagtaagcc tacgtcatat ccacctattt gttttttgtg gcctagggac attcaggaat 3600
ggaaagcccc actgattccc agagctagtt cattaagaag acagtgcctt tggtgggagc 3660
tatataagtt gtggctcttg gtgtgtgaac taactccttc aaggtaaatg aataggccta 3720
gatttattac tggggtgagc tggaagaaag gctcaggaag tgccaagctg tggctcaggt 3780
tactggaggg ctactatttg ctcaccaatg caaatgtatt agaagcaagc ttgtcaaata 3840
gtcatggaaa gaatgtgcag gaaaatcctt ctggagggaa aaatgggagc tgtgcattcc 3900
agaatttttt ctgcactgca cccagaggat gtagcccctg gaagtactta gatgcccatt 3960
gaaaaccacc tctttgtcct ataatctaga gagactcaca tgtgccttct tccgttcttt 4020
gagctagaag gtatttagga ttcagttaat cgtggttgct ataaaagttg cagcacctaa 4080
tgtatggcat aaataaatcc tttctgggaa gaaacacgga gctgcatttt tagagttcct 4140
tctccacact cctcccatag tatgaagtct gtggaagtgc atgcaggctc atataactgc 4200
cttttcctgt ggcctagaga gacttgcata cacctagtcc cctctacccc agagttggga 4260
ggtttaggat gcagtcctaa gagtggaaag agtggacaaa ctcttttcag gtcggattaa 4320
tagacctgca attatcactg gggttagggg agaaagcacg ggaagagaca atctccttct 4380
caggctgcta gtgggatatt tgtctccttt ctccccagtg catgttggaa accaggccac 4440
caagtagcca ctggaacagt gtgccataaa cccattccgg aggaagtgac agacagctgc 4500
attttaaagc cccttctgta cattgctact gggggataag gctcctggaa atgctatgca 4560
cctgtataaa atcacctttt tcctgtggtc tagagagact tgcatatgtc taattccctc 4620
tacttcaaga ggtaggaggt tgaggatgta gtcttaggtg gaagctgtaa tagtgggatg 4680
ctcatcatgt ggacaaactc taggaggaat cagtagacac aaaattatag ctgactagat 4740
tgggagaggc agttgtctca tctaacatac agaaactaac acagaaagtc aaagaaaatg 4800
atgaaacaga gatatatatt ccaattaaaa aacaaaataa atttccgaaa ctgtacccaa 4860
gtgatgtgga gatatgcaat ttacctgaca gggaattaga ataaggtcat aaagatgctc 4920
tctgatcagg tgaccaatgt aggaacaaaa ctgtgaattt caacaaagag attttttaaa 4980
gtttttaaaa caccagacga aattatagaa ctgaaaaata ctctgactaa aaaatctaat 5040
agagatgttc aacagcaggc tacatcaaac agaagaaaga atcagggaac tcaaagaaca 5100
ggtcattgaa agttatcaag tttctgtttc aagaaccaga aataccattt gacccagcaa 5160
tccattactg ggtatataca caaaggaata taaatcattc tgttctaaag acacatacat 5220
gggtatgttc actgcagcac tattcgcaat agcagagatg tggaatcaat ctaaatgccc 5280
atcaatgata cactggataa agaaaatgtg gtacatatac accatggaat actatgcagc 5340
tataagaaag aatgagatca tgtcctttgc agaaacatgg atgaagctgg aggccattat 5400
ccttagcaaa ccaatgcaga aacagaaaac caaataccac atgttcttac ttgtaagtgg 5460
gagctaaatg atgagaacac atggacacat agaggggaac aactcacact ggggcctact 5520

ggggtgaaga gtaggaggag agagagaagc agaaaaaata aataatgcgt actaggctta 5580

ctacctgcgt gacaaagtta atctgcaaaa caaccccatg acacgagttt atctatataa 5640

caaacctgca catgttagcc cctgaactta aaataaaaat taaatttaaa aaataagatt 5700

aatatctgca tacaaatctt tgtttacagc ttgttatata ctgaattatg tctgctcccc 5760

caacattcat atgttaaagc ccaaagttat tgtgtttgga aatagggctt ttaggagata 5820

tttaaggtta aataaggtta taaacgtgga gtcttaatct gataggattg gtggctttat 5880

aagaaaaaga aaagagattg ctctctcccc agtgcagtta ccaaggaaag gccatgtgag 5940

aacatagcag gaagggcagc catctgtaac ctaaggaaag agatctgtca aaggacaaaa 6000

ctacaacaaa tgtaaagatc tcaattggct ttatctgcga ttctggaatc aggcaatact 6060

ccatttcata aaacagaact agtgctccaa tgagctgagc aaaaggggtt gttagtttta 6120

tagacagaga aggacggaag aaagcagaaa taacgaatgg aaagtagatt ggtcaaattt 6180

actttccttt taaggcagaa aaatagaaca ataactattc aagtaatctt aaggttactt 6240

caggttaagg gttaaaaaca gaaggaattt tatcatcatg cttattgaag attcaaactg 6300

gcctgtttaa gaaattggct gttatctctt tctatctttt attttaagag gcttcctcag 6360

ataccatgct ggtggaagaa ggggagtgcc acctttctac ggccaggtgg acataaatat 6420

ccaagttccc cattcaatct ccttgccatt caaggcaagg tacc 6464

<210> 2
<211> 32
<212> DNA
<213> Artificial

<220>
<223> forward primer

<400> 2
ccctccagtt aacaaataca gcactggcat gg 32

<210> 3
<211> 31
<212> DNA
<213> Artificial

<220>
<223> forward primer

<400> 3
cctccagtta acaaatacag cactggcatg g 31

<210> 4
<211> 30
<212> DNA
<213> Artificial

<220>
<223> forward primer

<400> 4
ctccagttaa caaatacagc actggcatgg 30

<210>  5
<211>  29
<212>  DNA
<213>  Artificial

<220>
<223>  forward primer

<400>  5
tccagttaac aaatacagca ctggcatgg                                          29


<210>  6
<211>  28
<212>  DNA
<213>  Artificial

<220>
<223>  forward primer

<400>  6
ccagttaaca aatacagcac tggcatgg                                           28


<210>  7
<211>  27
<212>  DNA
<213>  Artificial

<220>
<223>  forward primer

<400>  7
cagttaacaa atacagcact ggcatgg                                            27


<210>  8
<211>  26
<212>  DNA
<213>  Artificial

<220>
<223>  forward primer

<400>  8
agttaacaaa tacagcactg gcatgg                                             26


<210>  9
<211>  25
<212>  DNA
<213>  Artificial

<220>
<223>  forward primer

<400>  9
gttaacaaat acagcactgg catgg                                              25


<210>  10
<211>  24
<212>  DNA
<213>  Artificial

<220>
<223>  forward primer

<400>  10
ttaacaaata cagcactggc atgg                                              24


<210>  11
<211>  23
<212>  DNA
<213>  Artificial

<220>
<223>  forward primer

<400>  11
taacaaatac agcactggca tgg                                               23


<210>  12
<211>  22
<212>  DNA
<213>  Artificial

<220>
<223>  forward primer

<400>  12
aacaaataca gcactggcat gg                                                22


<210>  13
<211>  21
<212>  DNA
<213>  Artificial

<220>
<223>  forward primer

<400>  13
acaaatacag cactggcatg g                                                 21


<210>  14
<211>  20
<212>  DNA
<213>  Artificial

<220>
<223>  forward primer

<400>  14
caaatacagc actggcatgg                                                   20


<210>  15
<211>  24
<212>  DNA
<213>  Artificial

<220>
<223>  reverse primer

<400>  15
gccacatctg ggaggagctt ccag                                              24


<210>  16
<211>  23
<212>  DNA
<213>  Artificial

<220>
<223>  reverse primer

<400>  16
ccacatctgg gaggagcttc cag                                               23


<210>  17
<211>  22
<212>  DNA
<213>  Artificial

<220>
<223>  reverse primer

<400>  17
cacatctggg aggagcttcc ag                                                22


<210>  18
<211>  21
<212>  DNA
<213>  Artificial

<220>
<223>  reverse primer

<400>  18
acatctggga ggagcttcca g                                                 21


<210>  19
<211>  20
<212>  DNA
<213>  Artificial

<220>
<223>  reverse primer

<400>  19
catctgggag gagcttccag                                                   20


<210>  20
<211>  19
<212>  DNA
<213>  Artificial

<220>
<223>  reverse primer

<400>  20
atctgggagg agcttccag                                                    19


<210>  21
<211>  18
<212>  DNA
<213>  Artificial

<220>
<223>  reverse primer

<400>  21
tctgggagga gcttccag                                                     18


<210>  22
<211>  17

<212>  DNA
<213>  Artificial

<220>
<223>  reverse primer

<400>  22
ctgggaggag cttccag                                                    17


<210>  23
<211>  34
<212>  DNA
<213>  Artificial

<220>
<223>  forward primer

<400>  23
tttcttaatt ctcatctcct gccaaagaag aaac                                 34


<210>  24
<211>  33
<212>  DNA
<213>  Artificial

<220>
<223>  forward primer

<400>  24
ttcttaattc tcatctcctg ccaaagaaga aac                                  33


<210>  25
<211>  32
<212>  DNA
<213>  Artificial

<220>
<223>  forward primer

<400>  25
tcttaattct catctcctgc caaagaagaa ac                                   32


<210>  26
<211>  31
<212>  DNA
<213>  Artificial

<220>
<223>  forward primer

<400>  26
cttaattctc atctcctgcc aaagaagaaa c                                    31


<210>  27
<211>  30
<212>  DNA
<213>  Artificial

<220>
<223>  forward primer

<400>  27
ttaattctca tctcctgcca aagaagaaac                                      30

<210>  28
<211>  29
<212>  DNA
<213>  Artificial

<220>
<223>  forward primer

<400>  28
taattctcat ctcctgccaa agaagaaac                                         29


<210>  29
<211>  28
<212>  DNA
<213>  Artificial

<220>
<223>  forward primer

<400>  29
aattctcatc tcctgccaaa gaagaaac                                          28


<210>  30
<211>  27
<212>  DNA
<213>  Artificial

<220>
<223>  forward primer

<400>  30
attctcatct cctgccaaag aagaaac                                           27


<210>  31
<211>  26
<212>  DNA
<213>  Artificial

<220>
<223>  forward primer

<400>  31
ttctcatctc ctgccaaaga agaaac                                            26


<210>  32
<211>  25
<212>  DNA
<213>  Artificial

<220>
<223>  forward primer

<400>  32
tctcatctcc tgccaaagaa gaaac                                             25


<210>  33
<211>  24
<212>  DNA
<213>  Artificial

<220>
<223>  forward primer

<400>  33
ctcatctcct gccaaagaag aaac                                              24


<210>  34
<211>  23
<212>  DNA
<213>  Artificial

<220>
<223>  forward primer

<400>  34
tcatctcctg ccaaagaaga aac                                               23


<210>  35
<211>  22
<212>  DNA
<213>  Artificial

<220>
<223>  forward primer

<400>  35
catctcctgc caaagaagaa ac                                                22


<210>  36
<211>  21
<212>  DNA
<213>  Artificial

<220>
<223>  forward primer

<400>  36
atctcctgcc aaagaagaaa c                                                 21


<210>  37
<211>  20
<212>  DNA
<213>  Artificial

<220>
<223>  forward primer

<400>  37
tctcctgcca aagaagaaac                                                   20


<210>  38
<211>  40
<212>  DNA
<213>  Artificial

<220>
<223>  reverse primer

<400>  38
ggaacaaaat gatgtggtta taaatgaaga tgttggagac                             40


<210>  39
<211>  39
<212>  DNA
<213>  Artificial

<220>
<223>  reverse primer

<400>  39
gaacaaaatg atgtggttat aaatgaagat gttggagac                              39


<210>  40
<211>  38
<212>  DNA
<213>  Artificial

<220>
<223>  reverse primer

<400>  40
aacaaaatga tgtggttata aatgaagatg ttggagac                               38


<210>  41
<211>  37
<212>  DNA
<213>  Artificial

<220>
<223>  reverse primer

<400>  41
acaaaatgat gtggttataa atgaagatgt tggagac                                37


<210>  42
<211>  36
<212>  DNA
<213>  Artificial

<220>
<223>  reverse primer

<400>  42
caaaatgatg tggttataaa tgaagatgtt ggagac                                 36


<210>  43
<211>  35
<212>  DNA
<213>  Artificial

<220>
<223>  reverse primer

<400>  43
aaaatgatgt ggttataaat gaagatgttg gagac                                  35


<210>  44
<211>  34
<212>  DNA
<213>  Artificial

<220>
<223>  reverse primer

<400>  44
aaatgatgtg gttataaatg aagatgttgg agac                                   34


<210>  45
<211>  33

<212>  DNA
<213>  Artificial

<220>
<223>  reverse primer

<400>  45
aatgatgtgg ttataaatga agatgttgga gac                                    33


<210>  46
<211>  32
<212>  DNA
<213>  Artificial

<220>
<223>  reverse primer

<400>  46
atgatgtggt tataaatgaa gatgttggag ac                                     32


<210>  47
<211>  31
<212>  DNA
<213>  Artificial

<220>
<223>  reverse primer

<400>  47
tgatgtggtt ataaatgaag atgttggaga c                                      31


<210>  48
<211>  30
<212>  DNA
<213>  Artificial

<220>
<223>  reverse primer

<400>  48
gatgtggtta taaatgaaga tgttggagac                                        30


<210>  49
<211>  29
<212>  DNA
<213>  Artificial

<220>
<223>  reverse primer

<400>  49
atgtggttat aaatgaagat gttggagac                                         29


<210>  50
<211>  28
<212>  DNA
<213>  Artificial

<220>
<223>  reverse primer

<400>  50
tgtggttata aatgaagatg ttggagac                                          28

<210>  51
<211>  27
<212>  DNA
<213>  Artificial

<220>
<223>  reverse primer

<400>  51
gtggttataa atgaagatgt tggagac                                          27


<210>  52
<211>  26
<212>  DNA
<213>  Artificial

<220>
<223>  reverse primer

<400>  52
tggttataaa tgaagatgtt ggagac                                           26


<210>  53
<211>  25
<212>  DNA
<213>  Artificial

<220>
<223>  reverse primer

<400>  53
ggttataaat gaagatgttg gagac                                            25


<210>  54
<211>  37
<212>  DNA
<213>  Artificial

<220>
<223>  forward primer

<400>  54
gctgagaaat atatttaaga tttccttggg gagaaat                               37


<210>  55
<211>  36
<212>  DNA
<213>  Artificial

<220>
<223>  forward primer

<400>  55
ctgagaaata tatttaagat ttccttgggg agaaat                                36


<210>  56
<211>  35
<212>  DNA
<213>  Artificial

<220>
<223>  forward primer

<400>  56
tgagaaatat atttaagatt tccttgggga gaaat                                35

<210>  57
<211>  34
<212>  DNA
<213>  Artificial

<220>
<223>  forward primer

<400>  57
gagaaatata tttaagattt ccttggggag aaat                                 34

<210>  58
<211>  33
<212>  DNA
<213>  Artificial

<220>
<223>  forward primer

<400>  58
agaaatatat ttaagatttc cttggggaga aat                                  33

<210>  59
<211>  32
<212>  DNA
<213>  Artificial

<220>
<223>  forward primer

<400>  59
gaaatatatt taagatttcc ttggggagaa at                                   32

<210>  60
<211>  31
<212>  DNA
<213>  Artificial

<220>
<223>  forward primer

<400>  60
aaatatattt aagatttcct tggggagaaa t                                    31

<210>  61
<211>  30
<212>  DNA
<213>  Artificial

<220>
<223>  forward primer

<400>  61
aatatattta agatttcctt ggggagaaat                                      30

<210>  62
<211>  29
<212>  DNA
<213>  Artificial

<220>
<223>  forward primer

<400>  62
atatatttaa gatttccttg gggagaaat                                         29


<210>  63
<211>  28
<212>  DNA
<213>  Artificial

<220>
<223>  forward primer

<400>  63
tatatttaag atttccttgg ggagaaat                                          28


<210>  64
<211>  27
<212>  DNA
<213>  Artificial

<220>
<223>  forward primer

<400>  64
atatttaaga tttccttggg gagaaat                                           27


<210>  65
<211>  26
<212>  DNA
<213>  Artificial

<220>
<223>  forward primer

<400>  65
tatttaagat ttccttgggg agaaat                                            26


<210>  66
<211>  25
<212>  DNA
<213>  Artificial

<220>
<223>  forward primer

<400>  66
atttaagatt tccttgggga gaaat                                             25


<210>  67
<211>  24
<212>  DNA
<213>  Artificial

<220>
<223>  forward primer

<400>  67
tttaagattt ccttggggag aaat                                              24


<210>  68
<211>  23

<212>  DNA
<213>  Artificial

<220>
<223>  forward primer

<400>  68
ttaagatttc cttggggaga aat                                              23


<210>  69
<211>  22
<212>  DNA
<213>  Artificial

<220>
<223>  forward primer

<400>  69
taagatttcc ttggggagaa at                                               22


<210>  70
<211>  21
<212>  DNA
<213>  Artificial

<220>
<223>  forward primer

<400>  70
aagatttcct tggggagaaa t                                                21


<210>  71
<211>  36
<212>  DNA
<213>  Artificial

<220>
<223>  reverse primer

<400>  71
tgataattag tgagttgggt gatacataca caaggg                                36


<210>  72
<211>  35
<212>  DNA
<213>  Artificial

<220>
<223>  reverse primer

<400>  72
gataattagt gagttgggtg atacatacac aaggg                                 35


<210>  73
<211>  34
<212>  DNA
<213>  Artificial

<220>
<223>  reverse primer

<400>  73
ataattagtg agttgggtga tacatacaca aggg                                  34

<210>  74
<211>  33
<212>  DNA
<213>  Artificial

<220>
<223>  reverse primer

<400>  74
taattagtga gttgggtgat acatacacaa ggg                                    33


<210>  75
<211>  32
<212>  DNA
<213>  Artificial

<220>
<223>  reverse primer

<400>  75
aattagtgag ttgggtgata catacacaag gg                                     32


<210>  76
<211>  31
<212>  DNA
<213>  Artificial

<220>
<223>  reverse primer

<400>  76
attagtgagt tgggtgatac atacacaagg g                                      31


<210>  77
<211>  30
<212>  DNA
<213>  Artificial

<220>
<223>  reverse primer

<400>  77
ttagtgagtt gggtgataca tacacaaggg                                        30


<210>  78
<211>  29
<212>  DNA
<213>  Artificial

<220>
<223>  reverse primer

<400>  78
tagtgagttg ggtgatacat acacaaggg                                         29


<210>  79
<211>  28
<212>  DNA
<213>  Artificial

<220>
<223>  reverse primer

<400>  79
agtgagttgg gtgatacata cacaaggg                                          28


<210>  80
<211>  27
<212>  DNA
<213>  Artificial

<220>
<223>  reverse primer

<400>  80
gtgagttggg tgatacatac acaaggg                                           27


<210>  81
<211>  26
<212>  DNA
<213>  Artificial

<220>
<223>  reverse primer

<400>  81
tgagttgggt gatacataca caaggg                                            26


<210>  82
<211>  25
<212>  DNA
<213>  Artificial

<220>
<223>  reverse primer

<400>  82
gagttgggtg atacatacac aaggg                                             25


<210>  83
<211>  24
<212>  DNA
<213>  Artificial

<220>
<223>  reverse primer

<400>  83
agttgggtga tacatacaca aggg                                              24


<210>  84
<211>  23
<212>  DNA
<213>  Artificial

<220>
<223>  reverse primer

<400>  84
gttgggtgat acatacacaa ggg                                               23


<210>  85
<211>  22
<212>  DNA
<213>  Artificial

<220>
<223>  reverse primer

<400>  85
ttgggtgata catacacaag gg                                                22


<210>  86
<211>  21
<212>  DNA
<213>  Artificial

<220>
<223>  reverse primer

<400>  86
tgggtgatac atacacaagg g                                                 21


<210>  87
<211>  19
<212>  DNA
<213>  Artificial

<220>
<223>  probe

<400>  87
tcctgccgtc agtggtcac                                                    19


<210>  88
<211>  18
<212>  DNA
<213>  Artificial

<220>
<223>  probe

<400>  88
cctgccgtca gtggtcac                                                     18


<210>  89
<211>  17
<212>  DNA
<213>  Artificial

<220>
<223>  probe

<400>  89
ctgccgtcag tggtcac                                                      17


<210>  90
<211>  16
<212>  DNA
<213>  Artificial

<220>
<223>  probe

<400>  90
tgccgtcagt ggtcac                                                       16


<210>  91
<211>  15

<212>  DNA
<213>  Artificial

<220>
<223>  probe

<400>  91
gccgtcagtg gtcac                                                        15


<210>  92
<211>  14
<212>  DNA
<213>  Artificial

<220>
<223>  probe

<400>  92
ccgtcagtgg tcac                                                         14


<210>  93
<211>  27
<212>  DNA
<213>  Artificial

<220>
<223>  probe

<400>  93
cttgaacctc aaacaattga agatttt                                           27


<210>  94
<211>  26
<212>  DNA
<213>  Artificial

<220>
<223>  probe

<400>  94
cttgaacctc aaacaattga agattt                                            26


<210>  95
<211>  25
<212>  DNA
<213>  Artificial

<220>
<223>  probe

<400>  95
cttgaacctc aaacaattga agatt                                             25


<210>  96
<211>  24
<212>  DNA
<213>  Artificial

<220>
<223>  probe

<400>  96
cttgaacctc aaacaattga agat                                              24

<210>  97
<211>  23
<212>  DNA
<213>  Artificial

<220>
<223>  probe

<400>  97
cttgaacctc aaacaattga aga                                              23


<210>  98
<211>  22
<212>  DNA
<213>  Artificial

<220>
<223>  probe

<400>  98
cttgaacctc aaacaattga ag                                               22


<210>  99
<211>  21
<212>  DNA
<213>  Artificial

<220>
<223>  probe

<400>  99
cttgaacctc aaacaattga a                                                21


<210>  100
<211>  20
<212>  DNA
<213>  Artificial

<220>
<223>  probe

<400>  100
cttgaacctc aaacaattga                                                  20


<210>  101
<211>  19
<212>  DNA
<213>  Artificial

<220>
<223>  probe

<400>  101
cttgaacctc aaacaattg                                                   19


<210>  102
<211>  18
<212>  DNA
<213>  Artificial

<220>
<223>  probe

<400>  102
cttgaacctc aaacaatt                                                    18


<210>  103
<211>  21
<212>  DNA
<213>  Artificial

<220>
<223>  probe

<400>  103
cccaaacctg gtgatgaatc c                                                21


<210>  104
<211>  20
<212>  DNA
<213>  Artificial

<220>
<223>  probe

<400>  104
ccaaacctgg tgatgaatcc                                                  20


<210>  105
<211>  19
<212>  DNA
<213>  Artificial

<220>
<223>  probe

<400>  105
caaacctggt gatgaatcc                                                   19


<210>  106
<211>  18
<212>  DNA
<213>  Artificial

<220>
<223>  probe

<400>  106
aaacctggtg atgaatcc                                                    18


<210>  107
<211>  17
<212>  DNA
<213>  Artificial

<220>
<223>  probe

<400>  107
aacctggtga tgaatcc                                                     17


<210>  108
<211>  16
<212>  DNA
<213>  Artificial

<220>
<223>  probe

<400>  108
acctggtgat gaatcc                                                        16


<210>  109
<211>  38
<212>  DNA
<213>  Artificial

<220>
<223>  forward primer

<400>  109
agaatttctt aattctcatc tcctgccaaa gaagaaac                                38


<210>  110
<211>  37
<212>  DNA
<213>  Artificial

<220>
<223>  forward primer

<400>  110
gaatttctta attctcatct cctgccaaag aagaaac                                 37


<210>  111
<211>  36
<212>  DNA
<213>  Artificial

<220>
<223>  forward primer

<400>  111
aatttcttaa ttctcatctc ctgccaaaga agaaac                                  36


<210>  112
<211>  35
<212>  DNA
<213>  Artificial

<220>
<223>  forward primer

<400>  112
atttcttaat tctcatctcc tgccaaagaa gaaac                                   35


<210>  113
<211>  40
<212>  DNA
<213>  Artificial

<220>
<223>  forward primer

<400>  113
agtgctgaga aatatattta agatttcctt ggggagaaat                              40


<210>  114
<211>  39

<212>  DNA
<213>  Artificial

<220>
<223>  forward primer

<400>  114
gtgctgagaa atatatttaa gatttccttg gggagaaat                              39


<210>  115
<211>  38
<212>  DNA
<213>  Artificial

<220>
<223>  forward primer

<400>  115
tgctgagaaa tatatttaag atttccttgg ggagaaat                               38


<210>  116
<211>  13
<212>  DNA
<213>  Artificial

<220>
<223>  probe

<400>  116
cgtcagtggt cac                                                          13


<210>  117
<211>  19
<212>  DNA
<213>  Artificial

<220>
<223>  probe

<400>  117
tcctgccgtc aatggtcac                                                    19


<210>  118
<211>  18
<212>  DNA
<213>  Artificial

<220>
<223>  probe

<400>  118
cctgccgtca atggtcac                                                     18


<210>  119
<211>  17
<212>  DNA
<213>  Artificial

<220>
<223>  probe

<400>  119
ctgccgtcaa tggtcac                                                      17

<210>  120
<211>  16
<212>  DNA
<213>  Artificial

<220>
<223>  probe

<400>  120
tgccgtcaat ggtcac                                                       16


<210>  121
<211>  15
<212>  DNA
<213>  Artificial

<220>
<223>  probe

<400>  121
gccgtcaatg gtcac                                                        15


<210>  122
<211>  14
<212>  DNA
<213>  Artificial

<220>
<223>  probe

<400>  122
ccgtcaatgg tcac                                                         14


<210>  123
<211>  13
<212>  DNA
<213>  Artificial

<220>
<223>  probe

<400>  123
cgtcaatggt cac                                                          13